# EUROPEAN PATENT APPLICATION

(11) **EP 4 282 432 A1**
(43) Date of publication of application: **29.11.2023**
(21) Application number: 21920747.9
(22) Date of filing: 29.11.2021
(51) Int. Cl.: A61K 47/38, A61K 47/26, A61K 47/10, A61K 9/08, A61K 45/00, A61P 27/02, A61P 31/12, A61P 29/00, A61P 31/02, A61P 31/04, A61P 31/10, A61P 27/10, A61P 27/06, A61P 37/02, A61P 35/00, A61P 9/10

(54) **CARRIER OR AUXILIARY MATERIAL OF OPHTHALMIC PREPARATION, PREPARATION METHOD THEREFOR, AND APPLICATION THEREOF**

(30) Priority: 22.01.2021 CN 202110091088
(71) Applicant: Chengdu Ruimu Biopharmaceuticals Co., Ltd., Chengdu, Sichuan 611135 (CN)
(72) Inventor: DONG, Qing, Chengdu, Sichuan 611135 (CN); XUE, Lubing, Chengdu, Sichuan 611135 (CN); TANG, Xin, Chengdu, Sichuan 611135 (CN)
(74) Representative: Altmann Stößel Dick Patentanwälte PartG mbB
(86) International application number: PCT/CN2021/134149
(87) International publication number: WO 2022/156371

(57) **Abstract**

A carrier or auxiliary material of an ophthalmic preparation, a preparation method therefor, and an application thereof, relating to the field of ophthalmic drug delivery. The carrier or auxiliary material of the ophthalmic preparation contains components such as a surfactant and an ionic polymer, and also contains a solvent. Or, the carrier or auxiliary material of the ophthalmic preparation contains components of low-polymerization-degree povidone and medium-polymerization-degree povidone, and also contains a solvent. Spherical nanobodies are formed in the solvent by the component contained in the carrier or auxiliary material of the ophthalmic preparation, have a particle size of 1-100 nm, and can be self-assembled to form spherical nanospheres having a particle size of 10-2000 nm. The administration carrier can wrap a drug through an anterior segment to efficiently deliver the drug to a posterior segment to play a therapeutic action, therby achieving the purpose of treating diseases of the ocular fundus by means of eye drop administration, solving the technical problem that is always worked on but not resolved in the field of ophthalmic drug delivery, and achieving extremely excellent clinical application prospects and very positive social significance.

## Description

### Technical field

The present invention belongs to the field of ophthalmic drug delivery, and in particular relates to a carrier or auxiliary material of an ophthalmic preparation, a preparation method therefor, and an application thereof.

### Background technology

There are many patients with fundus diseases, and only in China, the number of patients has exceeded tens of millions. With the aging society and the popularity of electronic products, the incidence rate will increase year by year; common fundus diseases include diabetic Macular edema, diabetic retinopathy (DR), age-related macular diseases, retinal vein occlusion (RVO), pathological myopia, geographic atrophy, eye tumors, endophthalmitis, etc., which may lead to decreased vision or even blindness, seriously affecting people's quality of life. For example, about 6.8% of diabetics suffer from diabetic Macular edema (DME), which is the main cause of blindness in diabetics (Urias et al., Vision Research, V139:221-227, 2017; Mandal et al., Ocular delivery of proteins and peptides: Challenges and novel formulation approaches, Advanced Drug Delivery Reviews, 126: 67-95, 2018).

Drug therapy is the main treatment method and research trend for fundus diseases. However, because of the complex physiological structure and barrier in the eyes, it is very difficult for medicaments to enter the eyeball, especially the posterior segment of the eye, and thus it is hard to achieve the effective dose, thereby, that brings great difficulties in realizing an effective therapy. Finding an effective and safe medicament or method for the treatment of fundus diseases is the object that researchers in this field have been striving for.

There are usually three ways for clinical ophthalmic administration: (1) Conjunctival sac administration (eye drops): medicaments can diffuse and distribute to the iris and Ciliary body after entering the anterior chamber water through the cornea, but the barrier effect of the lens and vitreous membrane makes it difficult for drugs to enter the lens and vitreous body; (2) Eye injection administration: it includes subconjunctival injection, anterior chamber injection, vitreous injection, retroocular injection, and orbital injection. Injecting medication can make the medicament directly reach the treatment site, but injection is traumatic and poses potential risks, such as anterior chamber injection can cause pain, photophobia, tearing, anterior chamber turbidity, bleeding, corneal endothelial cell damage, traumatic cataracts, etc; vitreous injection can cause lens opacity, vitreous organization, retinal/optic nerve damage, etc; (3) Systemic administration includes oral administration and intravenous administration: medicaments in the body generally gather in the liver, kidney or lung, and are blocked by the blood retinal barrier (BRB), so the concentration reaching the eye tissue is lower. Meanwhile, the whole body, especially the main organs, suffer unnecessary side effects.

At present, in order to allow medicaments to pass through the eye barrier in clinical practice, technical means such as eyeball intravitreal injections (IVI) or implant (ophthalmic insert) are usually used to deliver medicaments to the vitreous body of the patients for treating posterior segment diseases (Pei-Xue Ling, "Ophthalmic Drugs and Preparation Techniques", China Light Industry Press, 2010, P3; Wang et al., Mediators of Inflammation, Vol. 2013, Article ID 780634; Luaces-Rodríguez et al., Pharmaceutics, 2018, 10, 66). The operation for intravitreal injection or implant of medicaments is traumatic, that requires specially trained ophthalmologists to perform in sterile environments such as operating rooms; due to the traumatic nature of the operation, complications may occur, such as high intraocular pressure, cataracts, iatrogenic infectious endophthalmitis, vitreous hemorrhage, retinal damage, etc; the requirements of operating conditions and environment are high, and the operation must be carried out in hospitals where the conditions are permitted; the production and usage costs of biopharmaceutical eye injections are high; at the same time, for treatment opportunity, there are also cases of delayed treatment due to medical conditions, resulting in poor flexibility in adjusting dosage regimen (M. HATA et al., RETINA,37: 1320-1328, 2017).

Conjunctival sac administration is the most convenient and safe way of eye medication. However, the cornea has a multi-layer structure, that is roughly divided from the outside to the inside into: an epithelial layer rich in liposomes, a stromal layer rich in water-based components, and an endothelial layer rich in liposomes. After eye drops, the eye drops first contact with the tear layer on the surface of the eye, and then need to cross the epithelial layer, stromal layer, and endothelial layer to reach the posterior segment of the eye. During this process, due to the dilution of tears, the ocular surface barrier of the cornea and conjunctiva, and the anatomical position of the lens and vitreous body, eye drops often have high concentrations in the tissues of the anterior segment, and it is difficult for the eye drop to enter the posterior segment and achieve effective therapeutic concentrations. Therefore, although the way of conjunctival sac administration is safe, its drug delivery is poor, and thus brings great difficulties in achieving the object of effectively treating fundus diseases.

Totally, the main drug delivery ways in the prior art for the treatment of fundus diseases are difficult to give consideration to both safety and effectiveness.

Compared with intravenous injection and intravitreal injection, the route of eye drop administration has significant advantages in safety and convenience. Inventing an ophthalmic preparation that can deliver medicaments to the posterior segment of the eye is a technical problem that urgently needs to be solved in clinical practice, and the invention will be of great clinical treatment value and social significance. However, due to the unique structure of the eyeball, a medicament must be able to pass through multiple barrier layers comprising water-soluble layers and lipid-soluble layers for several times, before reaching the posterior segment of the eye. Currently, researchers have made various experiments, but none of them have achieved the desired effect.

Nanotechnology disperses medicaments to the nanoscale, gives them special physicochemical properties and different drug distribution and absorption characteristics, and thereby increases tissue and cell permeability, that may make medicaments have new therapeutic effects (T.L. Chang et al., Nanocrystal technology for drug formulation and delivery, Front. Chem. Sci. Eng. 2015, 9(1): 1-14; S. Jiang et al., Nanotechnology in retinal drug delivery, Int. J. Ophthalmol., 2018, 11(6): 1038-1044; M. Kabiri et al., A stimulus-responsive, in situ-forming, nanoparticle-laden hydrogel for ocular drug delivery, Drug Delivery and Translational Research, 2018, 8: 484-495; Cheng-Zheng Jia et al., Research Progress on Nanomaterials Penetrating Tissues and Cells, The Journal of New Industrialization, 2019, 9(9): 99-118). For example, the nanotechnology is used in the preparation of ophthalmic medicaments to increase the bioavailability and control drug delivery. Kassem et al. have prepared hydrocortisone nanosuspension (particle size: 0.539-4.87 µm) and other eye drops, which can increase intraocular pressure of rabbits (M.A. Kassem et al., Nanosuspension as an ophthalmic delivery system for certain glucocorticoid drugs, International J. Pharmaceutics, 340(2007): 126-133). It has been reported that cyclosporin A nanocapsules can enter the surface layer of the cornea in experimental animals, but they have not been able to enter the full layer of the cornea; for example, studies have shown that PEG-polycaprolactone nanoparticles can penetrate into the corneal epithelium after being dropped into rabbit eyes; it has also been found that subconjunctival and intravitreal injection of nanodrugs can control and delay drug release (Edited by Yi-Guang Jin, "Application of Nanotechnology in Drug Delivery", P319, Chemical Industry Press, 2015). Non-ionic surfactant vesicles (NSVs, also known as niosomes), that are composed of nonionic surfactant/nonionic amphiphilic compounds (such as Span, Poloxamer, Tween, etc.) and cholesterol as well as mostly have a diameter of submicron, are used as new drug delivery carriers. Bioadhesive materials can extend the residence time of vesicles in the eye, maintain sustained release, reduce drug clearance in the eye, and enhance corneal penetration. The bioavailability of medicaments by means of vesicles, respectively prepared with cyclopentolate (mydriatics), timolol maleate (antiglaucoma medicament) and acetazolamide (antiglaucoma medicament) has been improved in animal experiment (Xiao-Yu Zhao et al., Research progress on the application of novel drug carrier non-ionic surfactant vesicles, Chinese Hospital Pharmacy, 2008, 28(1): 833-5; X. Ge et al., Advances of Non Ionic Surfactant Vehicles (Neosomes) and Their Application in Drug Delivery, Pharmaceutics, 2019, 11, 55). Puras et al. injected the prepared cationic niosomes into the rat retina and subretina as a gene delivery mean (G Puras et al., A novel cationic niosome formulation for gene delivery to the retina, J. Control. Release, 2014, 174, 27-36). Although the use of nanotechnology in preparation of ophthalmic medicaments has certain advantages, the above research still fails to achieve the object of non-invasive eye drop administration that can deliver medicaments to the posterior segment of the eye and reach effective drug concentration.

Nano-Emulsion, also known as microemulsion, is composed of oil phase, water phase, surfactant and cosurfactant in a suitable ratio, and thus can have both hydrophilic and lipophilic properties, as well as have the potential to prepare a drug delivery system that can deliver medicaments to the posterior segment of the eye by eye drop administration. It is difficult for conventional nanoemulsion to have good hydrophilicity and lipophilicity at the same time, so that the nanoemulsion can pass through the tear layer on the surface of the eye, corneal epithelial cell layer, stroma and endothelial cell layer one by one and then deliver effective medicaments into the posterior segment of the eye. The oil phase of nanoemulsions often requires the use of vegetable oils, and the decomposition of oils may lead to the poor stability of the formulation; moreover, the amount of surfactants used is relatively large (ranging from 25% to 30%), which may cause toxic side effects and allergic reactions; cosurfactants such as ethanol may increase osmotic pressure, and are not suitable for eye drops, and are unstable (Edited by Yi-Guang Jin, "Application of Nanotechnology in Drug Delivery", P322, Chemical Industry Press, 2015; Wei-San Pan, Pharmaceutics, P404, Chemical Industry Press, 2017).

Therefore, investigating an eye drop delivery carrier for effectively transporting medicaments to the posterior segment of the eye is still an urgent and unresolved technical problem in this field.

### Summary of the invention

Aiming to address the problems mentioned above, the present invention provides an eye drop delivery carrier and the application thereof, which can efficiently transport medicaments to the posterior segment of the eye. The eye drop delivery carrier of the present invention can efficiently transport drugs to the posterior segment of the eye, and thus the present inventors have found a solution to the technical problems in the field of ophthalmic drug delivery that urgently needs to be settled but have not been successfully worked out.

The present invention provides a carrier or auxiliary material of an ophthalmic preparation, which contains the following components: a surfactant and an ionic polymer, and it also contains a solvent.

Further, the mass ratio of the surfactant to the ionic polymer is (1-100):(0.1-50); the ratio of the surfactant to the solvent is: every 100 mL of the solvent contains 5-3000 mg of the surfactant;
preferably, the mass ratio of the surfactant to the ionic polymer is (2.5-31):(1-7.5); the ratio of the surfactant to the solvent is: every 100 mL of the solvent contains 50-3000 mg of the surfactant.

Further, the surfactant is a non-ionic surfactant.

Further, the non-ionic surfactant is Spans, Polysorbates, Poloxamer, alkylglucosides, vitamin E polyethylene glycol succinate (TPGS), sucrose stearates or azone.

Further, the above-mentioned ionic polymer is selected from at least one of carboxymethyl cellulose (CMC) and its salts, sodium starch glycolate, hyaluronic acid and its salts, Xanthan gum, alginic acid and its salts, and polyethylene glycol diacetate PEG-(COOH)₂.

The present invention also provides a carrier or auxiliary material of an ophthalmic preparation, which contains the following components: a low-polymerization-degree povidone and a medium-polymerization-degree povidone, and it also contains a solvent.

Further, the mass ratio of the low-polymerization-degree povidone to the medium-polymerization-degree povidone is (0.1-10):1, and the ratio of the low-polymerization-degree povidone to the solvent is: every 100 mL of the solvent contains 5-3000 mg of the low-polymerization-degree povidone;
preferably, the mass ratio of the low-polymerization-degree povidone to the medium-polymerization-degree povidone is (0.24-1):1, and the ratio of the low-polymerization-degree povidone to the solvent is: every 100 mL of the solvent contains 400-840 mg of the low-polymerization-degree povidone.

More further, the low-polymerization-degree povidone mentioned above is a povidone with a weight average molecular weight of 2000-5000 Dalton, and preferably, is povidone PVP K12 with a weight average molecular weight of 3500 Dalton.

More further, the medium-polymerization-degree povidone is a povidone with a weight average molecular weight of 20000-60000 Dalton, and preferably, is povidone PVP K30 with a weight average molecular weight of 35000-50000 Dalton.

Further, in the carrier or auxiliary material of forementioned ophthalmic preparation, the solvent is a polar solvent.

More further, the polar solvent is water.

Further, the above-mentioned carrier or auxiliary material of an ophthalmic preparation also contains the following components: adhesive agents and/or cosolvents;
preferably, the adhesive agent is selected from at least one of polyethylene glycol, carbomer, Poloxamer, povidone, hydroxypropyl cellulose, methyl cellulose, hydroxyethyl cellulose, polyvinyl alcohol, Xanthan gum, polyoxyethylene fatty alcohols, hyaluronic acid and its salts or hydroxypropyl methyl cellulose (HPMC); the cosolvent is propylene glycol, glycerol, liquid polyethylene glycol, hydrogenated castor oil or castor oil; the mass ratio of the adhesive agent to the surfactant or the adhesive agent to the low-polymerization-degree povidone is 1:(0.1-100), and the mass ratio of the cosolvent to the surfactant or the cosolvent to the low-polymerization-degree povidone is (1-10): 1;
more preferably, the mass ratio of the adhesive agent to the surfactant or the adhesive agent to the low-polymerization-degree povidone is 1:(0.1-30).

Further, the carrier or auxiliary material of an ophthalmic preparation mentioned above also contains nanobodies, which are spherical and have a particle size of 1-100 nm; the nanobodies are formed by self-assembly of the components contained in the carrier or auxiliary material of the ophthalmic preparation.

Further, the nanobodies have a particle size of 5-30 nm.

More further, the carrier or auxiliary material of an ophthalmic preparation mentioned above also contains nanospheres, which are spherical in shape and have a particle size of 10-2000 nm; the nanospheres are formed by self-assembly of nanobodies.

More further, the particle size of the nanospheres is 100-2000 nm.

The present invention also provides a method for preparing the carrier or auxiliary material of an ophthalmic preparation mentioned above, which involves uniformly mixing the component and the solvent into a solution, and then the solution is ground or uniformly dispersed.

The present invention also provides the use of the carrier or auxiliary material of an ophthalmic preparation mentioned above in the preparation of carriers for eye drop administration, which can deliver the medicament to the posterior segment of eyes.

The present invention also provides an ophthalmic preparation for eye drop administration, which is a preparation composed of the carrier or auxiliary material of an ophthalmic preparation mentioned above as well as an active pharmaceutical ingredient for treating eye diseases.

Further, the mass ratio of the surfactant in the carrier or auxiliary material of an ophthalmic preparation mentioned above to the active pharmaceutical ingredient for treating eye diseases is (1-30):(1-2);
or, the mass ratio of the low-polymerization-degree povidone in the carrier or auxiliary material of an ophthalmic preparation mentioned above to the active pharmaceutical ingredient for treating eye diseases is (6-40): 1.

Further, the ophthalmic preparation carrier or auxiliary material mentioned above contains nanobodies, and the nanobodies are assembled with the active pharmaceutical ingredient for treating eye diseases.

More further, the nanobody is spherical, with a particle size of 1-100 nm; preferably, the particle size is 5-30 nm.

Further, the active pharmaceutical ingredient for treating eye diseases include small molecule compound medicaments, or free acids thereof, or free bases thereof, or pharmaceutically acceptable salts thereof;
preferably, the small molecule compound medicaments include nucleoside antiviral medicaments, intraocular pressure (IOP) lowering medicaments, antibiotics, antioxidant medicaments, anti-inflammatory medicaments, muscarinic receptor blocker medicaments, immunosuppressive medicaments, and glucocorticoid medicaments;
more preferably, the nucleoside antiviral medicaments include ganciclovir, aciclovir, penciclovir, cidofovir, fomivirsen, and lobucavir;
the IOP-lowering medicaments include a carbonic anhydrase inhibitor, and more preferably, are brinzolamide, acetazolamide, and methazolamide;
the antibiotics include amikacin, ceftriaxone, cefazolin, oxacillin, levofloxacin, ciprofloxacin, moxifloxacin, and vancomycin;
the anti-inflammatory medicament includes oxytetracycline;
the antioxidants include taurine, anthocyanidin, and lignin;
the muscarinic receptor blocker medicaments include atropine, scopolamine, and anisodamine;
the immunosuppressive medicaments include cyclosporine, tacrolimus, sirolimus, everolimus, mycophenolate mofetil, methotrexate, azathioprine, and cyclophosphamide;
the glucocorticoid medicaments include cortisone, prednisone, prednisolone, methylprednisolone, triamcinolone, and triamcinolone acetonide.

The present invention also provides a method for preparing the ophthalmic preparation mentioned above, which comprises the following steps:
(1) The surfactant and/or the adhesive agent is added to the solvent to prepare a solution;
(2) The active pharmaceutical ingredient for treating eye diseases and/or the cosolvent is dispersed in the solution obtained in step (1), to which is then added the ionic polymer or its solution, and the resultant solution is dispersed and mixed to obtain the initial suspension;
(3) The initial suspension obtained in step (2) is stirred and dispersed or homogenized to obtain the preparation;
or comprises the following steps:
(a) The low-polymerization-degree povidone and/or the adhesive agent is added to the solvent to prepare a solution;
(b) The active pharmaceutical ingredient for treating eye diseases and/or the cosolvent is dispersed in the solution obtained in step (a), to which is then added the medium-polymerization-degree povidone or its solution, and the resultant solution is dispersed and mixed to obtain the initial suspension;
(c) The mixed solution obtained in step (b) is ground or uniformly dispersed to obtain the preparation.

Further, the dispersion described in step (2) or step (b) is selected from at least one of mechanical stirring dispersion, magnetic stirring dispersion, vortex shaking dispersion, shear dispersion, homogeneous dispersion, grinding dispersion, and ultrasonic dispersion.

The present invention also provides the use of the ophthalmic preparation mentioned above in the preparation of medicaments for treating eye diseases.

Further, the ophthalmic preparation is an ophthalmic preparation for treating fundus diseases, and/or treating viral infectious diseases in the posterior segment of the eye, and/or treating chronic inflammation in the posterior segment of the eye, and/or reducing intraocular pressure, and/or relieving eye pain, and/or treating ocular bacterial or fungal infections, and/or preventing and treating juvenile myopia and pseudomyopia, and/or treating autoimmune diseases, and/or treating anterior segment diseases of the eye, and/or inhibiting tumor growth.

Further, the forementioned ophthalmic preparation for treating fundus diseases includes an ophthalmic preparation for treating macular edema, inflammatory edema, and inflammatory pain caused by fundus vascular diseases; more preferably, includes an ophthalmic preparation for treating macular edema due to central retinal vein occlusion, macular edema due to branch retinal vein occlusion, diabetic retinopathy (DR), diabetic macular edema (DME), pathological myopic macular edema, macular edema due to wet age-related macular degeneration, dry macular degeneration, geographic atrophy, endophthalmitis, acute retinal necrosis, postoperative inflammatory pain, and uveitis;
the ophthalmic preparation for treating viral infectious diseases in the posterior segment of the eye includes an ophthalmic preparation for treating cytomegaloviral uveitis, viral optic neuritis, and viral acute retinal necrosis;
the ophthalmic preparation for reducing intraocular pressure includes an ophthalmic preparation for treating acute and chronic glaucoma and its complications;
the ophthalmic preparation for treating autoimmune diseases includes an ophthalmic preparation for treating the eye diseases caused by ocular immune diseases (OID) or systemic autoimmune diseases; preferably, includes an ophthalmic preparation for treating Graves ophthalmopathy, Behoet's syndrome, birdshot retina choroidopathy, Sjogren's syndrome, sympathetic ophthalmia or granulomatous eye disease;
the ophthalmic preparation for treating anterior segment diseases of the eye includes an ophthalmic preparation for treating postoperative sequelae or complications of high-risk corneal transplantation, Vernal Kerato-Conjunctivits (VKC), Mooren's ulcer or spontaneous chronic corneal epithelial defects (SCCEDs), herpes simplex keratitis (HSK), corneal neovascularization or corneal pterygium.

Further, the active pharmaceutical ingredient of the ophthalmic preparation for treating intraocular bacterial or fungal infections is antibiotics;
the active pharmaceutical ingredient of the ophthalmic preparation for preventing and treating juvenile myopia and pseudomyopia is muscarinic receptor blockers;
the active pharmaceutical ingredient of the ophthalmic preparation for treating autoimmune diseases is mmunosuppressive agents.

The present invention also provides a method for treating eye diseases, that is, the forementioned ophthalmic preparation is administrated to the patients.

Further, the eye diseases are fundus diseases, and/or viral infectious diseases in the posterior segment of the eye, and/or chronic inflammation in the posterior segment of the eye, and/or ocular hypertension, and/or eye pain, and/or ocular bacterial or fungal infections, and/or juvenile myopia and pseudomyopia, and/or autoimmune diseases, and/or anterior segment diseases of the eye, and/or ocular tumors.

More further, the fundus diseases include macular edema, inflammatory edema, and inflammatory pain, that are caused by fundus vascular diseases; more preferably, are macular edema due to central retinal vein occlusion, macular edema due to branch retinal vein occlusion, diabetic retinopathy (DR), diabetic macular edema (DME), pathological myopic macular edema, macular edema due to wet age-related macular degeneration, dry macular degeneration, geographic atrophy, endophthalmitis, acute retinal necrosis, postoperative inflammatory pain, and uveitis;
the viral infectious diseases in the posterior segment of the eye include cytomegaloviral uveitis, viral optic neuritis, and viral acute retinal necrosis;
the ocular hypertension includes acute and chronic glaucoma and its complications;
the autoimmune diseases include the eye diseases caused by ocular immune diseases (OID) or systemic autoimmune diseases; preferably, include Graves ophthalmopathy, Behoet's syndrome, birdshot retina choroidopathy, Sjogren's syndrome, sympathetic ophthalmia or granulomatous eye disease;
the anterior segment diseases of the eye include postoperative sequelae or complications of high-risk corneal transplantation, Vernal Kerato-Conjunctivits (VKC), Mooren's ulcer or spontaneous chronic corneal epithelial defects (SCCEDs), herpes simplex keratitis (HSK), corneal neovascularization or corneal pterygium.

More further, the way used is eye drop administration.

The nanobody used in the present invention refers to a nanoscale spherical aggregate formed by self-assembly of the components of the ophthalmic preparation carrier or excipient in a solvent.

The nanosphere used in the present invention refers to spherical self-assembled structures formed by the self-assembly of nanobodies in a solvent.

The solvent used in the present invention refers to a liquid that can dissolve the components of an ophthalmic preparation carrier or excipient.

The surfactant used in the present invention refers to a substance that can significantly reduce the surface tension of a solution; the non-ionic surfactant used in the present invention refers to a surfactant that does not dissociate in water.

The ionic polymer used in the present invention refers to a polymer with cations or anions.

The low-polymerization-degree povidone used in the present invention refers to a povidone with a molecular weight of <10000 Dalton, while the medium-polymerization-degree povidone refers to a povidone with a molecular weight of >10000 Dalton but <100000 Dalton.

The "active pharmaceutical ingredient for treating eye diseases" used in the present invention refers to an Active Pharmaceutical Ingredient (API) that can be used for treating eye diseases, and has already been used as an ophthalmic medicament, as well as the mechanism and target of action indicate that this API can treat eye diseases, but has not been used as an ophthalmic medicament yet.

The eye drop administration described in the present invention refers to an administration method of dropping the drug solution into the eye, which belongs to the corneal route of administration.

Due to the unique nature of the eyeball structure, the drug delivery ways in the prior art can not satisfy the requirements of the safty and the efficient drug delivery, that is, the way that can efficiently deliver a medicament has safety issues, while the way that can perform the safe administration cannot transport effective medicaments. For example, the intravitreal injection can efficiently deliver a medicament, but there are serious complications such as intraocular bleeding and pain. At present, the eye drops are the safest, but due to the inability to pass through the anterior segment of the eye, it is difficult to provide a medicament to the posterior segment of the eye, and the effective concentration is not enough to realize effective treatment.

After years of experimentation, the inventors of the present application have developed the eyedrop drug delivery carrier of the present invention. After experimental verification, the carrier for eye drop administration can carry medicaments, transport medicaments to the vitreous body to play an action, and is stable, which provides a solution to the technical problems that are expected to be urgently settled but has not yet been overcome in the field of ophthalmic drug delivery.

Based on the experiments, the carrier of the present invention for eye drop administration can transport various drugs, which can achieve effective (expected) concentrations in the fundus of the eye, and do not affect the properties and effectiveness of the active pharmaceutical ingredients carried (encapsulated) for treating eye diseases. Predictably, the carrier for eye drop administration according to the present invention can be used to deliver the small molecule medicaments that are administered by intravitreal injection, intravitreal implant, oral administration and systemic injection, and thus can overcome the problems of intravitreal injection, intravitreal injection implant, oral administration and systemic injection in the prior art; solve the serious complications problems such as intraocular hemorrhage and pain; greatly reduce the pain of patients with fundus diseases; increase medical compliance; improve the life quality of patients and their families; or avoid systemic toxic and side effects caused by systemic medication.

The present invention can avoid complications caused by local injection or implant of the eye.

The preparation developed in the present invention has a small dosage as well as minimal toxic and side effects, and can not only be used as a therapeutic medicament, but also as a control and prevention for ophthalmic diseases.

The preparation of the present invention can meet the needs of long-term administration in clinical practice.

In the eye drop medication system of the present invention, the active pharmaceutical ingredient (API) can be a small molecule drug that has been used in clinical practice and has a clear mechanism of action. The quality is controllable, the product is easy to use, the patient has good compliance, and the doctor can flexibly adjust the medication scheme according to the patient's conditions.

Obviously, based on the above content of the present invention, according to the common technical knowledge and the conventional means in the field, without department from the above basic technical spirits, other various modifications, alternations, or changes can further be made.

With reference to the following specific examples of the embodiments, the above content of the present invention is further illustrated. But it should not be construed that the scope of the above subject matter of the present invention is limited to the following examples. The techniques realized based on the above content of the present invention are all within the scope of the present invention.

### Description of Figures

Figure 1. The transmission electron microscopy image of the sample prepared in Example 1 (with a scale of 200 nm) (A); the transmission electron microscopy image after staining with staining agent (with a scale of 1 µm) (B).

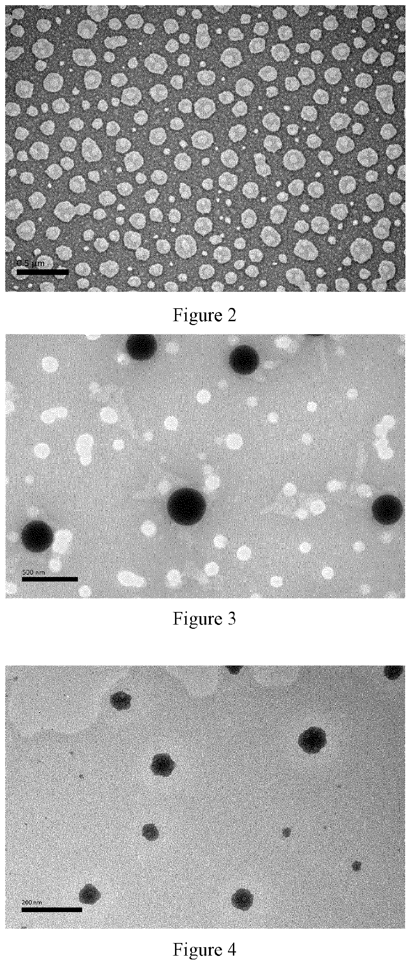

Figure 2. The transmission electron microscopy image of the sample prepared in Example 3 (with a scale of 0.5 µm).
Figure 3. The transmission electron microscopy image of the stained sample prepared in Example 6 (with a scale of 500 nm).
Figure 4. The transmission electron microscopy image of the stained sample prepared in Example 13 (with a scale of 200 nm).

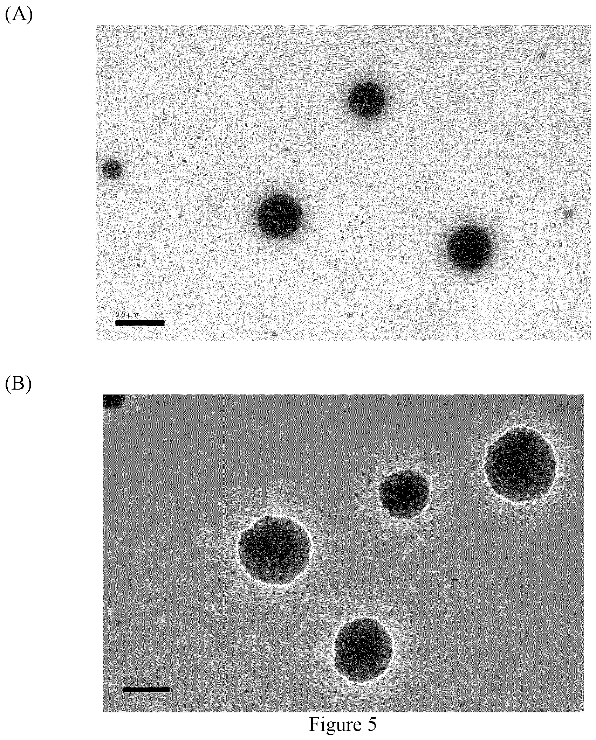

Figure 5. The transmission electron microscopy image of the sample prepared in Example 36 (with a scale of 0.5 µm) (A); the transmission electron microscopy image after adding the staining agent (with a scale of 0.5 µm) (B).

### Examples

The reagents or instruments used in the present invention can be obtained from market, and if specific conditions are not specified, they shall be used according to conventional conditions or manufacturer's recommended conditions.

Some instruments and equipment are as follows:
ES225SM-DR(E) electronic analytical balance, Precisa (Switzerland);
DF-101S Heat-collection type thermostatic magnetic agitator, Gongyi Yingyu Rivet Factory (Henan, China);
WH-2 Mini Vortex meter, Shanghai Huxi Analysis Instrument Factory (Shanghai, China);
Disperse machine: T25 easy clean digital, IKA (Germany);
KQ-500 type ultrasonic cleaning machine, Kunshan Ultrasonic Instruments Co., Ltd (Kunshan, China)
JP-010T type ultrasonic cleaning machine, Skymen Cleaning Equipment Shenzhen Co., Ltd.;
AH-NANO Plus High Pressure Homogenizer, AntosNanoTechnology (Suzhou) Co., Ltd. (China);
PM-DK2 Planetary Ball Mill, Droide Instrument and Equipment (Shanghai) Co., Ltd. (Shanghai, China);
Mettler Toledo FE20 pH meter, Mettler-Toledo (Switzerland);
NS-90 Nanoparticle Size Analyzer, Zhuhai Omec Instrument Co., Ltd (Zhuhai, China);
Agilent 1100 HPLC, Agilent Technologies Inc. (USA);
API4000 Triple Quadrupole Mass Spectrometer (Applied Biosystems, USA);
STY-1A Osmotic Pressure Measuring Instrument, Tiandatianfa Technology Corp., Ltd (Tianjin, China).

The method for detecting the properties of the preparation according to the present invention was as follows:

### Method for measuring particle size:

1 mL of sample prepared in the Example or Comparative Example was transferred to the sample cell. The detection temperature was set to 40 °C, and then the sample cell was put into the NS-90 Nanoparticle Size Analyzer to start the detection. Each sample was tested three times, and the particle size (in terms of light intensity distribution and % percentage) and polydispersity index (PdI) were represented with the average of three test results.

### Method for measuring osmotic pressure:

The osmotic pressure molar concentration of the solution was measured by measuring its freezing-point depression. Procedures: the probe of STY-1A osmotic pressure measuring instrument was cleaned. Three portions of 100 µL distilled water were respectively added to three sample tubes, and after preheating the instrument, the sample tube containing 100 µL of distilled water was screwed onto the instrument probe, followed by selecting "clean 3 times" and clicking "clean", that was repeated three times. Measuring: After filling in the sample information in the instrument information table, click "testing"; a pipette was used to transfer 100 µL of sample into the sample tube, which was gently screwed onto the instrument, followed by clicking "start" for testing. The test was repeated three times, and the average of the three test results was taken as the result.

### Method for measuring pH value:

The FE20 pH meter was calibrated using pH buffer solutions (pH 4.00, 6.86, and 9.18, respectively). The electrodes were rinsed with pure water, excess water was sucked off with fiber free paper, and then the electrodes were immersed in the liquid sample to be tested, followed by pressing the reading key to start the measuring. After the reading stabilized, the data obtained were the pH value of the sample.

If the pH value of the test solution was <5 or >9, the solution needed to be adjusted to pH 6~8 with acid or base. The commonly used pH regulators were NaOH and HCl, phosphoric acid and phosphate (e.g. sodium dihydrogen phosphate, disodium hydrogen phosphate), citric acid and citrate (e.g. sodium citrate), boric acid and borax; if the osmotic pressure of the obtained solution was detected not to reach an isotonic pressure, an appropriate amount of sodium chloride was added to make the solution reach or close to the isotonic.

Unless otherwise stated, the method for verifying the result of delivering medicaments to the posterior segment of the eye was as follows:
Experimental instrument and equipment: High performance liquid chromatography, model: LC-20AD (Shimadzu, Japan); mass spectrometer: model: API4000 triple quadrupole mass spectrometer (Applied Biosystems, USA); chromatographic column: Fortis Pace C18 5 µM, 2.1 × 30 mm (Fortis, UK).

Rats: Healthy adult Sprague Dawley (SD) rats were divided into a test group and a control group, with 6 eyes in each group. The test group received eye drops of the ophthalmic preparation prepared in the Example of the present invention, while the control group received the suspension of medicament (2 mg) in 5 mL of physiological saline (vortex shaking prior to use), at a dosage of 20 µL/eye. 0.5 h or 1 h after administration, the animals were euthanized, and the vitreous bodies were quickly collected. The vitreous samples were homogenized and stored at -80 °C. To 10 µL of vitreous homogenate, was added 90 µL of 95% ethanol, and then the solution was mixed under ultrasonic for 2 min, followed by vortex mixing for 1 min, to obtain the vitreous homogenate; to 50 µL of the homogenate, was added 175 µL of methanol, and then the resultant solution was vortex mixed for 3 min, and centrifuged at 4 °C and 12000 rpm for 10 min. The supernatant was filtered using a 0.45 µm needle filter. The filtrate was subjected to LC/MS/MS (Positive ion mode, MRM SCAN) analysis.

New Zealand rabbits: Healthy male New Zealand rabbits aged 3-4 months, weighing 2.0-2.5 kg, were selected and divided into two groups, with 4 eyes in each group. New Zealand rabbits were placed on an operating table, allowed to calm down, and then 30 µL of physiological saline was dropped into the eyes of animals in one group (blank control); 30 µL of test substance was dropped to the eyes of animals in another group, and after one hour, the animals were euthanized, then the vitreous bodies from both eyes were quickly collected and stored at -80 °C.

To the aqueous humor sample of New Zealand rabbits (50 µL), were added 50 µL of 75% acetonitrile-water and 150 µL of the internal standard (the solution of midazolam in acetonitrile (20 ng/mL)), and then the solution was vortex mixed for 10 min, centrifugated at 10000 rpm and 4 °C for 5 min. The supernatant was collected for LC-MS/MS analysis. After homogenizing the vitreous sample of New Zealand rabbits, 100 µL of sample solution was taken out and added with 100 µL of 75% acetonitrile-water and 50 µL of the internal standard (50 ng/mL of acetonitrile solution). The solution was vortex mixed for 10 min, and then centrifugated at 4 °C and 10000 rpm for 5 min. The supernatant was collected for LC-MS/MS (Positive ion mode, MRM SCAN) analysis.

### Example 1: Preparing the ophthalmic preparation according to the present invention

According to Table 1, 0.24 g of CMC Na (sodium carboxymethyl cellulose, ionic polymer) was weighed and added to a glass triangular flask containing 40 mL of purified water, and then stirred for 2 h with a magnetic stirrer to obtain solution 1; 1.0 g of polysorbate 80 (surfactant) and 0.24 g of HPMC (hydroxypropyl methyl cellulose, adhensive agent) were respectively weighed and transferred into a glass triangular flask containing 60 mL of pure water, and then stirred with a magnetic stirrer and heated in an water bath at 40 °C for about 1.5 h, to obtain solution 2; 40 mg of dexamethasone and 4 mL of PEG400 (i.e. 4.3 times the amount of surfactant (w/w)) were added into solution 2, and then the resultant solution was further heated and stirred for 30 min, which was then added to solution 1, followed by stirring for 30 min, to obtain the mixed solution; the mixed solution was dispersed with a disperser for 5 min at a speed of 9500 rpm, the disperser was turned off, and the solution was rested until the foam disappeared, followed by filtering under reduced pressure with a Büchner funnel, to obtain the dispersed solution; the dispersed solution was transferred to a high-pressure homogenizer, and homogenized at the temperature of 15 ± 5 °C under the pressure of about 400 Bar for 3 min, then homogenized under the increased pressure of >800 Bar for 25 min, followed by under the reduced pressure of 300 Bar for 2 min. The solution was discharged to obtain a colorless and clear solution, which was further filtered under reduced pressure, so as to remove bacteria and mechanical impurities, and obtain a colorless and clear solution after removal of impurities.
Adjusting pH value and osmotic pressure: 700 mg of NaH₂PO₄ and 400 mg of Na₂HPO₄ were added to adjust pH = 6.3; sodium chloride was added to adjust osmotic pressure to be 282 mOsmol/kg.
HPLC detection: instrument Agilent 1100 high-performance liquid chromatography; operating software: OpenLab CDS c.01.10 (201) Chemstation Edition;
Chromatographic conditions: the chromatographic column was Waters XBridge C18 5 µm, 4.6 × 250 mm; column temperature 35 °C, flow rate 1.0 mL/min, detection wavelength 240 nm; mobile phase: 0.1% phosphoric acid aqueous solution (72.0%) - acetonitrile (28.0%) isocratic elution. The sample was diluted with the mobile phase in a ratio of 1:5, and then 10 µL of sample solution was injected into HPLC, with a test result of 96.2%.

The particle size was 20.6 nm (85.6%), and PdI was 0.266. The sample was stored at room temperature in dark for 1 month, and the appearance and the content was not changed.

0.5 h after eye drops, the concentration of dexamethasone in the vitreous body of animals was 53.4 (ng/g), with an RSD of 36.6%. The animals in the control group was dropped with a suspension of 2 mg dexamethasone/5 mL physiological saline (votex shaking prior to use), 20 µL for each eye. 0.5 h after eye drops, no dexamethasone was detected in the vitreous body of animals in the control group (below the detection limit, < 1 ng/g).

### Example 2:

The preparation method was almost identical to that in Example 1, and the raw materials and their amounts were shown in Table 1. After removal of impurities, a colorless and clear solution was obtained.

Adjusting pH value: 0.2 N NaOH and/or 0.1 N HCl was added to adjust pH to be 6.5.

The HPLC detection method was the same as that in Example 1. The content result detected by HPLC: 95.1%, the particle size 12.9 nm (92.1%), PdI: 0.509; the stability was better, and there was no significant change in the appearance and the content after being stored in dark at room temperature for one month; a small amount of precipitation appeared after two months.

1 h after eye drops, the API concentration in the rat vitreum was 13.9 ng/g, with an RSD of 17.2%.

### Example 3:

The preparation method was almost identical to that in Example 1, and the raw materials and their amounts were shown in Table 1. After removal of impurities, a yellowish and clear solution was obtained, and then sodium chloride was added to adjust osmotic pressure to be 273 mOsmol/kg.
HPLC detection: column: ZORBAX Eclipse Plus C18, 4.6 × 100 mm, 3.5 µm; mobile phase A: 0.1% formic acid aqueous solution, mobile phase B: ACN. Temp.: 35 °C, detection wavelength 296 nm, flow rate 0.8 mL/min; gradient elution program: 0-2': 95% A-5% B, 15': 55% A-45% B, 18-21': 35% A-65% B, 23': 95% A-5% B. The detection result: 98.2%.
Particle size 13.2 nm (81.2%) and 57.7 nm (13.1%), PdI: 0.431; the stability was good, and after storing in dark at 40 °C for 20 days, no significant changes in the appearance and the content was observed;
0.5 h after eye drops, the API concentration in the rat vitreum was 315 ng/g, with an RSD of 29.4%.
0.5 h after eye drops, the API concentration in the vitreous body of New Zealand rabbits was 142 ng/g, with an RSD of 34.3%.

### Example 4:

The preparation was performed by referring to the method in Example 3, and after removal of impurities, a yellowish and clear solution was obtained. The raw materials and their amounts were shown in Table 1.

The detection result: 16.6 nm (96.2%), PdI: 0.229.

The HPLC detection method was the same as that in Example 3. The content result: 97.5%; the stability was good, and there was no obvious change in the appearance and the content after storing in dark at 40 °C for 20 days.

### Example 5:

The preparation method was almost identical to that in Example 1, and the raw materials and their amounts were shown in Table 1. After removal of impurities, a colorless and clear solution was obtained, and then 0.1 N NaOH was added to adjust pH to be 6.5.
HPLC detection: column: ZORBAX Eclipse Plus C18, 4.6 × 100 mm, 3.5 µm; mobile phase A: 40 mM ammonium acetate aqueous solution (pH 5.0), mobile phase B: MeOH. Temp.: 35 °C, detection wavelength 233 nm, flow rate 0.8 mL/min; gradient elution program: 0-2': 100% A, 20-22': 60% A-40% B, 23': 100% A. The detection result: 97.4%;
Particle size 11.8 nm (71.6%), PdI: 0.519. After storing in dark at room temperature for one month, no obvious changes in the appearance and the content was observed.

### Example 6:

The preparation was performed by referring to the method in Example 5, and the raw materials and their amounts were shown in Table 1. After removal of impurities, a colorless and clear solution was obtained, and then 1 N sodium citrate solution was added to adjust pH to be 6.5, while sodium chloride was added to adjust osmotic pressure to be 297 mOsmol/kg.
HPLC detection: Column: ZORBAX 300SB-CN, 2.1 × 150 mm, 5 µm; mobile phase: 40 mM KH₂PO₄ (pH 4.5):methanol (75:25) isocratric elution, Temp.: 35 °C, detection wavelength: 233 nm, flow rate: 0.8 mL/min; test result: 99.1%.
Particle size 21.6 nm (94.4%), PdI: 0.206. After storing in dark at room temperature for two months, no obvious changes in the appearance and the content was observed.

0.5 h after eye drops, the API concentration in the vitreous body of animals was 39.8 ± 16.6 ng/g.

### Example 7:

The preparation method was almost identical to that in Example 5, and the raw materials and their amounts were shown in Table 1. After removal of impurities, a colorless and clear solution was obtained. pH test result: 6.9, no adjustment required.

The HPLC detection method was the same as that in Example 5, with a detection result of 98.6%; particle size: 16.6 nm (98%); PdI: 0.227; no obvious change in the appearance and the content was observed after storing at room temperature in dark for 2 months.

### Example 8:

The preparation method was almost identical to that in Example 5, and the raw materials and their amounts were shown in Table 1. After removal of impurities, a colorless and clear solution was obtained. pH test result: 6.5, no adjustment required.

The HPLC detection method was the same as that in Example 5, with a detection result of 97.8%; particle size: 17.1 nm (55.5%), 513 (36.3%); PdI: 0.795; no obvious change in the appearance and the content was observed after storing at room temperature in dark for 1 month.

### Example 9:

Preparation method: 60 mg of CMC-Na was weighed and then added to a glass triangular flask containing 15 mL of pure water, and then stirred for 2 h with a magnetic stirrer to obtain solution 1; 0.24 g of polysorbate 80 and 0.12 g of HPMC (adhensive agent) were respectively weighed and then added into a glass triangular flask containing 15 mL of pure water, and then stirred with a magnetic stirrer and heated in an water bath at 40 °C for about 3 h, to obtain solution 2; 15 mg of lipoic acid and 1 mL of glycerol (equivalent to 5.25 times the amount of surfactant (w/w)) were added into solution 2, and then the resultant solution was further heated and stirred for 30 min. Subsequently, the solution was added to solution 1, and then stirred for 30 min, to obtain the mixed solution; the mixed solution was dispersed with a disperser for 3 min at a speed of 11,000 rpm, the disperser was turned off, and the solution was rested until the foam disappeared, followed by transferring to a high-pressure homogenizer for homogenization treatment (for conditions, referring to Example 1), to obtain a colorless clear solution. Then, the solution was filtered under reduced pressure to remove bacteria and mechanical impurities, and thus a colorless and clear solution was obtained after removal of impurities;
Method for adjusting pH value and osmotic pressure: 0.1N sodium citrate solution was added to adjust pH to be 6.3; sodium chloride was added to adjust osmotic pressure to be 294 mOsmol/kg.
HPLC detection: column: ZORBAX Eclipse Plus C18, 4.6 × 100 mm, 3.5 µm; mobile phase A: 0.1% phosphoric acid (pH 3.0), B: methanol-acetonitrile (1:1). Temp.: 35°C, detection wavelength: 215 nm, flow rate: 0.8 mL/min; gradient elution program: 0-5': 60% A-40% B, 28-30': 40% A-60% B; detection result: 97.4%. Particle size: 17.8 nm (98.6%); PdI: 0.222; no obvious change in the appearance and the content was observed after storing at 3-8 °C in dark for 1 month.

0.5 h after eye drops, the API concentration in the vitreum of rats was 52.6±17.9 ng/g.

In the control group, 2 mg of lipoic acid/5mL of normal saline suspension (vortex shaking prior to use) was added dropwise, 20 µL for each eye. In the control group, lipoic acid was not detected in the vitreum of animals 0.5 h after eye drops (below the detection limit, <1 ng/g).

### Example 10:

The preparation method and the method for adjusting pH value and osmotic pressure were almost identical to that in Example 5, and the raw materials and their amounts were shown in Table 1. After removal of impurities, a colorless and clear solution was obtained.

The HPLC detection method was the same as that in Example 5, with a detection result of 98.4%.

Detection results: particle size 348 nm (85%), PdI: 0.422.

No obvious change in the appearance and the content was observed after storing at room temperature in dark for 1 month; there was no significant change after 1 month, but a small amount of precipitation appeared after 2 months.

### Example 11:

The preparation method and the methods for adjusting pH value and osmotic pressure were almost identical to that in Example 9, and the raw materials and their amounts were shown in Table 1, wherein the dosage of the cosolvent propylene glycol was 6.2 times that of the surfactant (w/w).

Except that the HPLC detection wavelength was 233 nm, the detection method was the same as that in Example 9, with a detection result of 98.1%; particle size 25.8 nm (87.4%), PdI: 0.317; no obvious change in the appearance and the content was observed after storing at 3-8 °C in dark for 1 month.

### Example 12:

The preparation method and the methods for adjusting pH value and osmotic pressure were almost identical to that in Example 9, and the raw materials and their amounts were shown in Table 1, wherein the dosage of the cosolvent propylene glycol was 8.9 times that of the surfactant (w/w).

The HPLC detection method was the same as that in Example 9, with a detection result of 95.2%; particle size 31.5 nm (82.9%), PdI: 0.347; no obvious change in the appearance and the content was observed after storing at 3-8 °C in dark for 1 month.

### Example 13:

The preparation method was almost identical to that in Example 1, and the raw materials and their amounts were shown in Table 1. After removal of impurities, a yellowish and clear solution was obtained.
Adjusting pH value and osmotic pressure: 0.1 N NaOH was added to adjust pH to be 6.3; sodium chloride was added to adjust osmotic pressure to be 297 mOsmol/kg;
The detection wavelength used in HPLC was 280 nm, and the remained were the same as that in Example 1, with a detection result of 97.3%; particle size 16.7 nm (98.1%), PdI: 0.225; no change in the appearance and the content was observed after storing at room temperature in dark for 1 month.

0.5 h after eye drops, the API concentration in the vitreum of rats was 66.5 ± 18.1 ng/g.

In the control group, 2 mg of doxycycline/5mL of normal saline suspension (vortex shaking prior to use) was administrated by eye drops, 20 µL for each eye. In the control group, doxycycline was not detected in the vitreum of animals 0.5 h after eye drops (below the detection limit, <1 ng/g).

### Example 14:

The preparation method and the methods for adjusting pH value and osmotic pressure were almost identical to that in Example 13, and the raw materials and their amounts were shown in Table 1. After removal of impurities, a yellowish and clear solution was obtained.

The detection method by HPLC was the same as that in Example 13, with a HPLC detection result of 98.2%; particle size 17.2 nm (97.9%), PdI: 0.208; no change in the appearance and the content was observed after storing at room temperature in dark for 1 month.

### Example 15:

The preparation method and the methods for adjusting pH value and osmotic pressure were almost identical to that in Example 13. The addition of cosolvent was as follows: 1.5 mL of propylene glycol (equivalent to 10 times the amount of surfactant (w/w)) was weighed and added, together with the surfactant, to the medium water; the resultant solution was dissolved by magnetic stir and heating in a water bath to obtain solution 2; after removal of impurities, a yellowish and clear solution was obtained. The raw materials and their amounts were shown in Table 1.

The HPLC detection method was the same as that in Example 13, with a detection result of 95.2%; particle size 29.7 nm (89.3%), PdI: 0.382; the cottony substance appeared after storing at 3-8 °C in dark for 1 month.

### Example 16:

The preparation method and the methods for adjusting pH value and osmotic pressure were almost identical to that in Example 9, and the raw materials and their amounts were shown in Table 1.

The HPLC detection method was the same as that in Example 9, with a detection result of 0.486 mg/mL (metformin) and 0.481 mg/mL (lipoic acid); particle size 18.9 nm + 302.1 nm, PdI: 0.529; there was no change in the appearance and the content after storing at 3-8 °C in dark for 1 month.

0.5 h after eye drops, the API concentration in the vitreum of animals was 86.5 ng/g (lipoic acid) and 69.5 ng/g (metformin).

### Example 17:

The preparation method and the methods for adjusting pH value and osmotic pressure were almost identical to that in Example 9, and the raw materials and their amounts were shown in Table 1.

The HPLC detection method was the same as that in Example 9, with a detection result of 0.487 mg/mL (doxycycline) and 0.478 mg/mL (lipoic acid); particle size 20.2 nm + 251.6 nm, PdI: 0.701; no change in the appearance and the content was observed after storing at 3-8 °C in dark for 1 month.

0.5 h after eye drops, the API concentration in the vitreum of animals was 57.3 ng/g (lipoic acid) and 68.4 ng/g (doxycycline).

### Example 18:

The preparation method was almost identical to that in Example 1, and the raw materials and their amounts were shown in Table 1. After removal of impurities, a colorless and clear solution was obtained, and then sodium chloride was added to adjust osmotic pressure to be 301 mOsmol/kg; pH test result: 6.6, no adjustment required.

HPLC detection: column: ZORBAX Eclipse Plus C18, 4.6 × 100 mm, 3.5 µm; mobile phase A: 0.1% phosphoric acid, B: acetonitrile, Temp.: 35°C, detection wavelength: 260 nm, flow rate: 0.8 mL/min; gradient elution program: 0-2': 95% A-5% B, 20-25': 65% A-35% B, 28': 95% A-5% B; detection result: 98.2%. Particle size: 20.3 nm (83.6%); PdI: 0.249; no obvious change in the appearance and the content was observed after storing at room temperature for 1 month.

1 h after eye drops, the API concentration in the vitreum of New Zealand rabbits was 138 ng/g, while the API concentration in the aqueous humor was 681 ng/g.

### Example 19:

The preparation method and the methods for adjusting pH value and osmotic pressure were almost identical to that in Example 1, and the raw materials and their amounts were shown in Table 1. After removal of impurities, a yellowish and clear solution was obtained.

HPLC detection: column: ZORBAX Eclipse Plus C18, 4.6 × 100 mm, 3.5 µm; mobile phase A: 0.1% phosphoric acid, B: methanol (80:20), Temp.: 35°C, detection wavelength: 280 nm, flow rate: 0.8 mL/min; detection result: 98.4%. Particle size: 39.7 nm (95.5%); PdI: 0.318; no obvious change in the appearance and the content was observed after storing at room temperature for 1 month.

0.5 h after eye drops, the API concentration in the vitreum of rats was 78.3 ng/g.

### Example 20:

The preparation method and the methods for adjusting pH value and osmotic pressure were almost identical to that in Example 19, and the raw materials and their amounts were shown in Table 1. After removal of impurities, a yellowish and clear solution was obtained.

The detection method by HPLC was the same as that in Example 19, with a detection result of 97.8%; particle size 46.2 nm (95.5%), PdI: 0.343; no change in the appearance and the content was observed after storing at room temperature in dark for 1 month.

### Example 21:

The preparation method was almost identical to that in Example 1, and the raw materials and their amounts were shown in Table 1. After removal of impurities, a colorless and clear solution was obtained, and then sodium chloride was added to adjust osmotic pressure to be 271 mOsmol/kg; pH test result: 6.6, no adjustment required.

HPLC detection: column: ZORBAX Eclipse Plus C18, 4.6 × 100 mm, 3.5 µm; mobile phase: 0.1% H₃PO₄-acetonitrile (85:15) isocratic elution, Temp.: 35°C, detection wavelength: 217 nm, flow rate: 0.7 mL/min; detection result: 99.6%. Particle size: 15.2 nm (93.4%); PdI: 0.227; no obvious change in the appearance and the content was observed after storing at room temperature in dark for 1 month.

0.5 h after eye drops, the API concentration in the vitreum of rats was 79.4 ng/g.

### Example 22:

The preparation was performed by referring to the method in Example 21, and the raw materials and their amounts were shown in Table 1. After removal of impurities, a colorless and clear solution was obtained, and then sodium chloride was added to adjust osmotic pressure to be 265 mOsmol/kg; pH test result: 6.5, no adjustment required.

The detection method by HPLC was the same as that in Example 21, with a detection result of 98.3%; particle size 11.9 nm (91.9%), PdI: 0.206; no change in the appearance and the content was observed after storing at room temperature in dark for 1 month.

0.5 h after eye drops, the API concentration in the vitreum of rats was 46.2 ng/g.

### Example 23:

The preparation was performed by referring to the method in Example 9, and the raw materials and their amounts were shown in Table 1, wherein the amount of the cosolvent propylene glycol was 4.5 times that of the surfactant (w/w). pH value was 6.5, close to the isotonic, and thus there was no need to adjust.
HPLC detection method: Column: ZORBAX Eclipse Plus C18, 4.6 × 100 mm, 3.5 µm; mobile phase A: 0.1% phosphoric acid, B: acetonitrile (80:20), isocratic elution; Temp.: 35 °C, detection wavelength: 306 nm, flow rate: 0.8 mL/min; detection result: 95.7%;
Particle size 27.5 nm (77.9%), PdI: 0.328; no change in the appearance and the content was observed after storing at 3-8 °C in dark for 1 month.
0.5 h after eye drops, the API concentration in the vitreum of rats was 20.3 ± 9.3 ng/g.

### Example 24:

The preparation was performed by referring to the method in Example 23, and the raw materials and their amounts were shown in Table 1, wherein the amount of the cosolvent propylene glycol was 4.5 times that of the surfactant (w/w). After removal of impurities, a colorless and clear solution was obtained.
pH value was 6.6, and thus there was no need to adjust; adjusting osmotic pressure: sodium chloride was added to adjust osmotic pressure to be 293 mOsmol/kg;
The detection method by HPLC was the same as that in Example 14, with a detection result of 96.1%;
particle size 24.5 nm (85.5%), PdI: 0.253; no change in the appearance and the content was observed after storing at 3-8 °C in dark for 1 month.

### Example 25:

The preparation was performed by referring to the method in Example 23, and the raw materials and their amounts were shown in Table 1, wherein the amount of the cosolvent propylene glycol was 4.5 times that of the surfactant (w/w). After removal of impurities, a colorless and clear solution was obtained.
pH value was 6.4, and thus there was no need to adjust; adjusting osmotic pressure: sodium chloride was added to adjust osmotic pressure to be 305 mOsmol/kg;
The detection method by HPLC was the same as that in Example 14, with a detection result of 94.7%;
particle size 26.2 nm (75.2%), PdI: 0.325; no change in the appearance and the content was observed after storing at 3-8 °C in dark for 1 month.

### Example 26:

The preparation was performed by referring to the method in Example 23, and the raw materials and their amounts were shown in Table 1, wherein the amount of the cosolvent propylene glycol was 4.5 times that of the surfactant (w/w). After removal of impurities, a colorless and clear solution was obtained.
Adjusting pH value and osmotic pressure: 0.2 N NaOH was added to adjust the pH to 6.2; sodium chloride was added to adjust osmotic pressure to be 305 mOsmol/kg;
The detection method by HPLC was the same as that in Example 14, with a detection result of 95.3%;
particle size 22.7 nm (83.4%), PdI: 0.372; no change in the appearance and the content was observed after storing at 3-8 °C in dark for 1 month.

### Example 27:

The preparation method was almost identical to that in Example 1, and the raw materials and their amounts were shown in Table 1. After removal of impurities, a colorless and clear solution was obtained, and then 1M sodium citrate aqueous solution was added to adjust the pH to 6.2, while sodium chloride was added to adjust osmotic pressure to be 307 mOsmol/kg;
HPLC detection: column: ZORBAX Eclipse Plus C18, 4.6 × 100 mm, 3.5 µm; mobile phase A: water; mobile phase B: methanol; flow rate: 0.8 mL/min; gradient elution program: 0-10': 100% A-0% B, 15': 55% A-45% B, 18-21': 35% A-65% B; Temp.: 30 °C, detection wavelength: 255 nm, detection result of the purity: 99.2%; particle size 19.6 nm (75.9%); PdI: 0.424; no obvious change in the appearance and the content was observed after storing at room temperature in dark for 1 month.
0.5 h after eye drops, the API concentration in the vitreum of rats was 580 ng/g.

### Example 28:

The preparation method was almost identical to that in Example 9, and the raw materials and their amounts were shown in Table 1, wherein the amount of the cosolvent propylene glycol was 5 times that of the surfactant (w/w). After removal of impurities, a colorless and clear solution was obtained.
Adjusting pH value and osmotic pressure: 0.1 N NaOH was added to adjust pH to be 6.3; sodium chloride was added to adjust osmotic pressure to be 290 mOsmol/kg;
The HPLC detection was carried out by referring to the method in Example 9, with a detection result of 96.1%;
Particle size: 23.7 nm (84.2%); PdI: 0.323; no obvious change in the appearance and the content was observed after storing at room temperature in dark for 1 month.
0.5 h after eye drops, the API concentration in the vitreum of rats was 62.5 ng/g.

### Example 29:

The preparation method was almost identical to that in Example 1, and the raw materials and their amounts were shown in Table 1, wherein the amount of the cosolvent PEG400 was 5 times that of the surfactant (w/w). After removal of impurities, a colorless and clear solution was obtained.
Adjusting pH value and osmotic pressure: 1 M Na₂HPO₄ solution was added to adjust pH to be 6.2; sodium chloride was added to adjust osmotic pressure to be 295 mOsmol/kg;
The HPLC detection was carried out by referring to the method in Example 1, with a detection result of 97.3%; particle size: 22.4 nm (91.4%); PdI: 0.293; no obvious change in the appearance and the content was observed after storing at room temperature in dark for 1 month.
0.5 h after eye drops, the API concentration in the vitreum of rats was 42.7 ng/g.

### Example 30:

The preparation was performed by referring to the method in Example 9, and the raw materials and their amounts were shown in Table 1, wherein the amount of the cosolvent propylene glycol was 4.2 times that of the surfactant (w/w). After removal of impurities, a colorless and clear solution was obtained.
Adjusting pH value and osmotic pressure: 0.1 N NaOH was added to adjust pH to be 6.3; sodium chloride was added to adjust osmotic pressure to be 288 mOsmol/kg;
HPLC detection was carried out by referring to the method in Example 9, with a detection result of 95.6%; particle size: 24.1 nm (81.5%); PdI: 0.357; no obvious change in the appearance and the content was observed after storing at room temperature in dark for 1 month.

### Example 31:

The preparation was performed by referring to the method in Example 5, and the raw materials and their amounts were shown in Table 1, wherein the amount of the cosolvent propylene glycol was 5.2 times that of the surfactant (w/w). After removal of impurities, a colorless and clear solution was obtained.
Adjusting pH value and osmotic pressure: 0.2 N NaOH was added to adjust pH to be 6.3; sodium chloride was added to adjust osmotic pressure to be 310 mOsmol/kg;
HPLC detection was carried out by referring to the method in Example 5, with a detection result of 97.2%; particle size: 27.5 nm (79.6%); PdI: 0.364; visible particles were observed after storing at room temperature in dark for 1 month.

### Example 32:

The preparation method was almost identical to that in Example 1, and the raw materials and their amounts were shown in Table 1. After removal of impurities, a colorless and clear solution was obtained. 1 M sodium citrate solution was added to adjust pH to be 6.2; sodium chloride was added to adjust osmotic pressure to be 308 mOsmol/kg;
HPLC detection: Column: ZORBAX Eclipse Plus C18, 4.6 × 100 mm, 3.5 µm; mobile phase A: 0.1% phosphoric acid aqueous solution, mobile phase B: methanol. Temp.: 35 °C, detection wavelength: 280 nm, flow rate: 0.8 mL/min; gradient elution procedure: 0-2': 85% A-15% B, 15-20': 35% A-65% B, 22-25': 15% A-85% B; test result: 95.3%; particle size 17.6 nm (93.9%), PdI: 0.229; after storing at room temperature in dark for 1 month, no obvious change in the appearance and the content was observed.
0.5 h after eye drops, the API concentration in the vitreum of rats was 185.3 ng/g.

### Example 33:

The preparation was performed by referring to the method in Example 9, and the raw materials and their amounts were shown in Table 1, wherein the amount of the cosolvent castor oil was 3 times that of the surfactant.

Except that HPLC detection wavelength was 280 nm, the detection method was the same as that in Example 9, with a detection result of 94.7%; particle size 19.7 nm (86.4%); PdI: 0.331; pH 6.8; no obvious change in the appearance and the content was observed after storing at room temperature in dark for 1 month.

0.5 h after eye drops, the API concentration in the vitreum of rats was 37.6 ng/g.

### Example 34

The preparation was performed by referring to the method in Example 1, and the raw materials and their amounts were shown in Table 1. After removal of impurities, a colorless and clear solution was obtained. pH 6.5, no adjustment required;
Except that HPLC detection wavelength was 245 nm, the detection conditions were the same as that in Example 1, with a detection result of 98.1%; particle size 18.6 nm (96.9%); PdI: 0.257; no obvious change in the appearance and the content were observed after storing at room temperature in dark for 1 month.
0.5 h after eye drops, the API concentration in the vitreum of rats was 43.8 ng/g.

### Example 35:

The raw materials and their amounts were shown in Table 1, and the preparation was performed by referring to the method in Example 1. After removal of impurities, a colorless and clear solution was obtained.
Detection results: particle size 18.6 nm (96.5%), PdI: 0.218;
Detection result of the content by HPLC: 97.2%;
no obvious change in the appearance and the content were observed after storing at room temperature in dark for 1 month.
0.5 h after eye drops, the API concentration in the vitreum of rats was 5.1 ng/g.

### Example 36:

The raw materials and their amounts were shown in Table 1, and the preparation was performed by referring to the method in Example 1. The amount of the cosolvent PEG400 added was equal to that of the surfactant (1: 1 (w/w)). After removal of impurities, a colorless and clear solution was obtained.

Detection results: particle size 19.6 nm (97.0%), PdI: 0.289; pH 6.33, osmotic pressure: 275 mOsmol/kg; the detection result of the content by HPLC: N/A.

No obvious change in the appearance and the content was observed after storing at room temperature in dark for 1 month.

### Comparative example 1

The preparation and the detection were performed by referring to the method in Example 1, wherein CMC-Na was substituted with the low-polymerization-degree povidone, and the raw materials and their amounts were shown in Table 1.

Detection results: particle size 10.0 nm (98.8%), PdI: 0.363, stability: precipitation formed after storing in a cool place for 1 month.

### Comparative example 2

The preparation and the detection were performed by referring to the method in Example 1, and the raw materials and their amounts were shown in Table 1.

Detection results: particle size 196 nm (61.6%), PdI: 0.828, stability: cottony precipitation formed after storing in a cool place for 1 month. The supernatant was collected to measure the particle size, with results: 530 nm (53.9%) and 225 nm (46.1%), PDI: 1.00.

### Example 37:

According to Table 2, 0.25 g of povidone (PVP) K30 was weighed and then transferred to a glass triangular flask containing 15 mL of pure water, and then stirred for 2 h with a magnetic stirrer to obtain solution 1; 60 mg of HPMC and 60 mg of povidone (PVP) K12 were respectively weighed and then added into a glass triangular flask containing 15 mL of pure water, and then stirred with a magnetic stirrer and heated in an water bath at 40 °C for about 3 h, to obtain solution 2; 36 mg of moxifloxacin hydrochloride was weighed and added into solution 2, and then the resultant solution was further heated and stirred for 30 min. Subsequently, the solution was added to solution 1, and then stirred for 30 min, to obtain the mixed solution; the mixed solution was subjected to the operations of disperse, high pressure homogenization and membrane filtration to remove bacteria and mechanical impurities, the same as that in Example 3. After removal of impurities, a yellowish and clear solution was obtained, and then sodium chloride was added to adjust osmotic pressure to be 285 mOsmol/kg;
Detection results: particle size 14.0 nm (54.2%), 222 nm (31.9%) and 10.1 nm (13.1%), PdI: 0.564; the detection result of the content by HPLC: 96.4%.
Stability: no obvious change in the appearance and the content was observed after storing at 40 °C for 20 days. The particle size increased, and the detection results were 242 nm (77.9%) and 18.1 nm (12.4%).

### Example 38:

The preparation was performed by referring to the method in Example 1, and the raw materials and their amounts were shown in Table 2, wherein the amount of the cosolvent PEG400 was 5 times that of the low-polymerization-degree povidone (w/w). After removal of impurities, a colorless and clear solution was obtained, with a pH value of 6.5, no adjustment required;
The HPLC detection method was the same as that in Example 1, with the detection result of 98.1%;
Particle size 15.1 nm (87.1%) and 3.1 nm (11.0%), PdI: 0.288; no obvious change in the appearance and the content were observed after storing at room temperature in dark for 1 month.
0.5 h after eye drops, the API concentration in the vitreum of rats was 5.1 ng/g.

### Example 39:

The raw materials and their amounts were shown in Table 2, and the preparation and the HPLC detection were performed by referring to the method in Example 21. pH test result: 6.68, no adjustment required.

HPLC detection result: 99.3%; particle size 11.5 nm (62.9%) and 77.8 nm (23.6%), PdI: 0.362; no obvious change in the appearance and the content were observed after storing at room temperature in dark for 1 month.

### Example 40:

The raw materials and their amounts were shown in Table 2, and the preparation was performed by referring to the method in Example 1. After removal of impurities, a colorless and clear solution was obtained.

HPLC detection was carried out by referring to the method in Example 1, with a detection result: 98.5%, particle size 125.6 nm (63.5%), 13.6 nm (33.1%), PdI: 0.255; a light emulsion with white precipitate formed after being placed in a dark place at room temperature for 1 month.

### Comparative example 3

The raw materials and their amounts were shown in Table 2, and the preparation was performed by referring to the method in Example 1, wherein the medium-polymerization-degree povidone was substituted with an ionic polymer CMC-Na, to obtain a light white emulsion.

Detection results: particle size 1299 nm, PdI: 0.175; after standing overnight, white precipitates generated.

After eye drop administration, the test results of API concentration in the vitreous body of rats indicated that the ophthalmic medicament of the present invention could carry the active pharmaceutical ingredients for treating eye diseases and cross the barrier of the eyeball structure, and thereby deliver the effective dose of medicaments to the vitreous body by means of conjunctival sac administration (eye drop administration), which could avoid invasive administration methods such as intravitreal injection, and also significantly reduce the total drug amount and the absorption of drugs in the whole body, so as to prevent toxic and side effects.

**Table 1**

| Examp le | Surfactant (A) | Ionic polymer (B) | Adhesive agent (C) | API (mg) (D) | A:B:C:D mass ratio | Cosolv ent | Solven t |
|---|---|---|---|---|---|---|---|
| 1 | Polysorbate 80 | Sodium carboxymeth yl cellulose (CMC-Na) | hydroxyprop yl methylcellul ose (HPMC) | Dexamethason e 40 mg | 25:6:6:1 | PEG4 00 | Water 100mL |
| 2 | Polysorbate 20 | CMC-Na | Carbomer 941 | Dexamethason e 10 mg | 30:5:1:1 | | 25 mL Water |
| 3 | Polysorbate 80 | CMC-Na | Povidone (PVP K12) | Moxifloxacin hydrochloride 36 mg | 8.3:2:2:1 | | 30 mL Water |
| 4 | Polysorbate 80 | CMC-Na | PVP K12 | Moxifloxacin hydrochloride 36 mg | 25:6:6:1 | | Water 30 mL |
| 5 | Polysorbate 80 | CMC-Na | Hydroxyprop yl cellulose (HPC) | Metformin hydrochloride 15 mg | 20:4:8:1 | | 30 mL Water |
| 6 | Polysorbate 80 | CMC-Na | HPMC | Metformin hydrochloride 15 mg | 20:4:7:1 | | 30 mL Water |
| 7 | Polysorbate 80 | CMC-Na | Poloxamer P-188 | Metformin hydrochloride 15 mg | 25:4:4:1 | | 30 mL Water |
| 8 | Polysorbate 80 | CMC-Na | HPC | Metformin hydrochloride 15 mg | 16:4:8:1 | | 30 mL Water |
| 9 | Polysorbate 80 | CMC-Na | HPMC | Lipoic acid 15 mg | 16:4:8:1 | Glycer ol | Water 30 mL |
| 10 | Poloxamer | Sodium alginate | PVP K12 | Metformin hydrochloride 15 mg | 1:3.5:10: 1 | | 30 mL Water |
| 11 | Polysorbate 80 | Sodium alginate | HPMC | Lipoic acid 15 mg | 16:4:5:1 | Propyl ene glycol | Water 30 mL |
| 12 | Lauryl glucoside | CMC-Na | HPC | Lipoic acid 15 mg | 14:4:8:1 | Glycer ol | Water 30 mL |
| 13 | Polysorbate 80 | CMC-Na | Poloxamer | Doxycycline 15 mg | 16:4:8:1 | | Water 30 mL |
| 14 | Polysorbate 80 | CMC-Na | HPMC | Doxycycline 15 mg | 16:4:8:1 | | Water 30 mL |
| 15 | TPGS (vitamin E polyethylene glycol succinate) | CMC-Na | HPMC | Doxycycline 15 mg | 10:4:6:1 | Propyl ene glycol | Water 30 mL |
| 16 | Polysorbate 80 | CMC-Na | Poloxamer | Lipoic acid 15 mg Metformin hydrochloride 15 mg | 24:6:14:(1: 1) | | Water 30 mL |
| 17 | Polysorbate 80 | CMC-Na | Poloxamer | Lipoic acid 15 mg Doxycycline 15 mg | 30:6:12:( 1:1) | | Water 30 mL |
| 18 | Polysorbate 80 | CMC-Na | Poloxamer | Brinzolamide 12 mg | 24:4:8:1 | | Water 30 mL |
| 19 | Polysorbate 80 | CMC-Na | PVPK12 | Diclofenac 30 mg | 12:4:6:1 | | Water 30 mL |
| 20 | Polysorbate 80 | Sodium hyaluronate | HPMC | Diclofenac 30 mg | 12:2:6:1 | | Water 30 mL |
| 21 | Polysorbate 80 | CMC-Na | Sodium hyaluronate | Atropine sulfate 4.5 mg | 25:6:5:1 | | Water 45 mL |
| 22 | Polysorbate 80 | CMC-Na | Poloxamer | Atropine sulfate 4.5 mg | 20:5:5:1 | | Water 90 mL |
| 23 | Polysorbate 80 | CMC-Na | Xanthan gum | Resveratrol 10mg | 16:4:5:1 | Propyl ene glycol | Water 25 mL |
| 24 | Polysorbate 80 | CMC-Na | HPC | Resveratrol 10 mg | 25:5:6:1 | Propyl ene glycol | Water 25 mL |
| 25 | Polysorbate 80 | Sodium starch glycolate | HPMC | Resveratrol 10 mg | 25:4:6:1 | Glycer ol | Water 25 mL |
| 26 | Polysorbate 80 | PEG2K-(CO OH)₂ | HPMC | Resveratrol 10mg | 25:5:6:1 | Propyl ene glycol | Water 25 mL |
| 27 | Polysorbate 80 | CMC-Na | HPMC | Ganciclovir 80 mg | 16.8:3:7. 5:1 | | Water 100 mL |
| 28 | Span 60 | CMC-Na | HPMC | Lipoic acid 15 mg | 22:5:6:1 | Propyl ene glycol | Water 30 mL |
| 29 | Span 40 | CMC-Na | HPMC | Dexamethason e 10 mg | 22:5:6:1 | PEG4 00 | Water 25 mL |
| 30 | Sucrose stearate | CMC-Na | Xanthan gum | Lipoic acid 15 mg | 2:5:6:1 | Propyl ene glycol | Water 30 mL |
| 31 | Azone | CMC-Na | Xanthan gum | Lipoic acid 15 mg | 2:5:6:1 | Propyl ene glycol | Water 30 mL |
| 32 | Polysorbate 80 | CMC-Na | HPMC | Vancomycin hydrochloride 40 mg | 25:5:6:1 | | Water 60 mL |
| 33 | Polysorbate 80 | CMC-Na | HPMC | Rapamycin 4 mg | 25:5:7:1 | Castor oil | Water 40 mL |
| 34 | Polysorbate 80 | CMC-Na | HPMC | Hydrocortison e 40 mg | 25:6:5:1 | | Water 100 mL |
| 35 | Polysorbate 80 | CMC-Na | HPMC | Betamethason e 4 mg | 31:7.5:7. 5:1 | PEG4 00 | Water 10 mL |
| 36 | Polysorbate 80 300 mg | CMC-Na 75 mg | HPMC 75 mg | N/A | 4:1:1 | 300 mg PEG4 00 | Water 25 mL |
| Comp arative examp le 1 | Polysorbate 80 | - | PVPK12 HPMC | Moxifloxacin hydrochloride 24 mg | A:C:D=2 5:6:1 | | Water 30 mL |
| Comp arative examp le 2 | - | Sodium hyaluronate | - | Atropine sulfate 4.5 mg | B:D=10: 1 | | Water 45 mL |

**Table 2**

| Exampl e | Low-polymer ization-degre e povidone (A) | Medium-poly merization-de gree povidone (B) | Adhensiv e agent (C) | API (mg) (D) | A:B:C:D mass ratio | Cosolv ent | Solvent |
|---|---|---|---|---|---|---|---|
| 37 | PVP K12 | PVPK30 | HPMC | Moxifloxacin hydrochloride 36 mg | 6:25:6:1 | | Water 30 mL |
| 38 | PVP K12 | PVP K30 | HPMC | Dexamethason e 20 mg | 21:26:13: 1 | PEG | Water 50 mL |
| 39 | PVP K12 | PVPK30 | HPMC | Atropine sulfate 4.5 mg | 40:80:20: 1 | | Water 45 mL |
| 40 | PVP K12 | PVPK30 | PEG4000 | Dexamethason e 10 mg | 15:15:5:1 | | Water 25 mL |
| Compar ative example 3 | PVP K12 | CMC-Na | PVP K30 | Dexamethason e 10 mg | 10:15:5:1 | | Water 25 mL |

In the following, the beneficial effects of the carrier or auxiliary material of an ophthalmic preparation according to the present invention were demonstrated with reference to the Experimental examples.

### Experimental example 1: Observation results of the carriers according to the present invention by transmission electron microscope

### Transmission Electron Microscope (JEM-2100 Plus, JEOL, Japan)

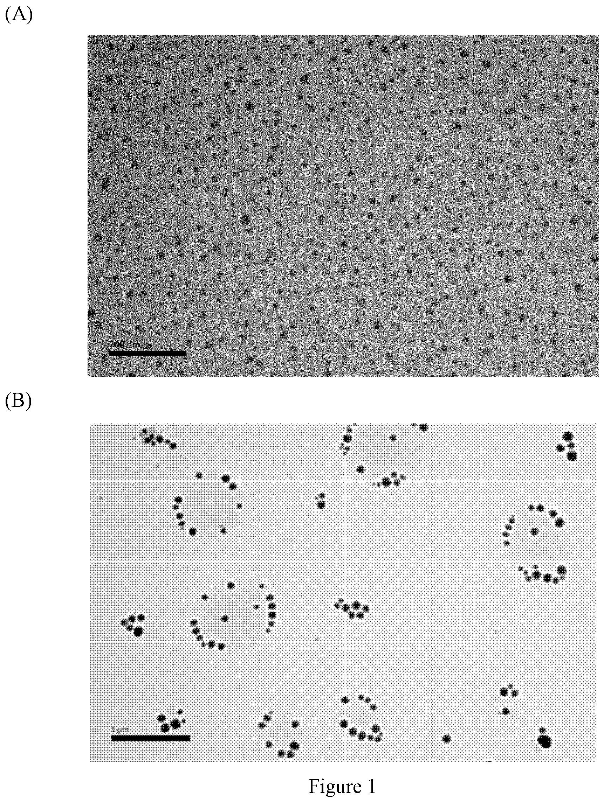

One drop of the solution sample prepared in Example 3-1 was transferred into the copper mesh, and after standing for 5 min, any excess liquid sample was removed, then the copper mesh was allowed to dry naturally and placed in the specimen chamber for testing; sample staining: one drop of liquid sample was transferred into the copper mesh, and after removing the excess sample from the sample mesh, one drop of 2% phosphomolybdic acid was added, followed by standing for 5 min. Then, the excess liquid was removed, and the mesh was naturally dried, and placed in the electron microscope for testing. The results are shown in Figure 1. It could be found that the drug carrier prepared in the present invention formed a spherical structure with a particle size of 1-100 nm in the solvent (nanobodys, Figure 1A), and the nanobodies can further self-assemble into spheres with a particle size of 10-2000 nm (nanospheres, Figure 1B).

### Experimental example 2: Detection of the particle size, the content, and the stability

### 1. Experimental methods

1 mL of sample prepared in the Example and the Comparative example was transferred to the sample cell, and the detection temperature was set to 40 °C. The sample cell was put into NS-90 nanoparticle size analyser for testing. Each sample was tested three times, and the average of the three test results was represented by the particle size (in terms of light intensity distribution and % percentage) and the polydispersity index (PdI). After testing, the sample was stored in dark, the appearance changes were observed, and then the particle size was retested.

The content of the ophthalmic preparation sample prepared in the present invention was detected by Agilent 1100 high-performance liquid chromatography.

### 2. Experimental results

See Tables 4 and 5:

**Table 4:**

| Exam ple | Initial particle size (percentage)/PdI/ HPLC content | Storing in a dark place at a certain temperature for one month (unless otherwise stated) | |
|---|---|---|---|
| | | Particle size (percentage)/PdI | Appearance and content changes |
| 1 | 20.6 nm (85.6%), PdI: 0.266 HPLC content: 96.2% | Room temperature, 20.2 nm (89.3%), PdI: 0.247 | No obvious changes were observed. |
| 2 | 12.9 nm (92.1 %), PdI: 0.509 HPLC content: 95.1% | Room temperature, 14.7 nm (86.2%), PdI: 0.325 | No obvious changes were observed after one month; a small of amount precipitations generated after two months. |
| 3 | 13.2 nm (81.2%) and 57.7 nm (13.1%), PdI: 0.431 HPLC content: 98.2% | Storing at 40 °C for 20 days: 14.8 nm (96.6%), PdI: 0.235 | No obvious changes were observed. |
| 4 | 16.6 nm (96.2%), PdI: 0.229 HPLC content: 97.5% | Storing at 40 °C for 20 days: 16.0 nm (98.8%), PdI: 0.168 | No obvious changes were observed. |
| 5 | 11.8 nm (71.6%), PdI: 0.519 HPLC content: 97.4% | Room temperature, 12.50 nm (96.2%), PdI: 0.318 ; HPLC content: 96.3% | No obvious changes were observed. |
| 6 | 21.6 nm (94.4%), PdI: 0.206 HPLC content: 99.1% | Storing at room temperature for 2 months, 20.2 nm (91.2%), PdI: 0.215 ; HPLC content: 97.3% | No obvious changes were observed. |
| 7 | 16.6 nm (98%), PdI: 0.227 HPLC content: 98.6% | Storing at room temperature for 2 months, 17.2 nm (92.0%), PdI: 0.215 | No obvious changes were observed. |
| 8 | 17.1 nm (55.5%) and 513 (36.3%), PdI: 0.795 HPLC content: 97.8% | Room temperature, 18.5 nm (82.5 %), PdI: 0.429, HPLC content: 95.3% | No obvious changes were observed. |
| 9 | 17.8 nm (98.6%), PdI: 0.222 HPLC content: 97.4% | 3-8 °C, 19.1 nm (94.2%), PdI: 0.317 | No obvious changes were observed. |
| 10 | 348 nm (85%), PdI: 0.422 HPLC content: 98.4% | Room temperature, 178 nm (94.2%), PdI: 0.317 | No obvious changes were observed after one month; a small amount precipitations generated after two months. |
| 11 | 25.8 nm (87.4%), PdI: 0.317 HPLC content: 98.1% | 3-8 °C, 23.6 nm (94.2%), PdI: 0.342; | No obvious changes were observed. |
| 12 | 31.5 nm (82.9%), PdI: 0.347 HPLC content: 95.2% | 3-8 °C, 29.5 nm (89.4%), PdI: 0.420 | No obvious changes were observed. |
| 13 | 16.7 nm (98.1%), PdI: 0.225 HPLC content: 97.3% | Room temperature, 17.3 nm (95.8%), PdI: 0.261 | No obvious changes were observed. |
| 14 | 17.2 nm (97.9%), PdI: 0.208; HPLC content: 98.2% | Room temperature, 18.4 nm (93.6%), PdI: 0.265 | No obvious changes were observed. |
| 15 | 29.7 nm (89.3%), PdI: 0.382; HPLC content: 95.2% | 3-8 °C, 31.4 nm (53.8%), 246 nm (36.7%), PdI: 0.541 | Cottony substances were observed. |
| 16 | 18.9 nm (45.3%) and 302.1 nm (43.9%), PdI: 0.529 HPLC content: 0.486 mg/mL (Metformin), 0.481 mg/mL (Lipoic acid) | 3-8 °C, 20.3 nm (44.6%), 243 nm (45.2%), PdI: 0.372 | No obvious changes were observed. |
| 17 | 20.2 nm (40.7%) and 251.6 nm (61.0%), PdI: 0.701 HPLC content: 0.487 mg/mL (Doxycycline), 0.478 mg/mL (Lipoic acid) | 3-8°C, 21.8 nm (42.1%) and 237 nm (57.0%), PdI: 0.472 | No obvious changes were observed. |
| 18 | 20.3 nm (83.6%), PdI: 0.249 HPLC content: 98.2% | Room temperature, 21.5 nm (90.2%), PdI: 0.225 | No obvious changes were observed. |
| 19 | 39.7 nm (95.5%), PdI: 0.318 HPLC content: 98.4% | Room temperature, 40.2 nm (92.7%), PdI: 0.258 | No obvious changes were observed. |
| 20 | 46.2 nm (95.5%), PdI: 0.343 HPLC content: 97.8% | Room temperature, 43.8 nm (91.4%), PdI: 0.226 | No obvious changes were observed. |
| 21 | 15.2 nm (93.4%), PdI: 0.227 HPLC content: 99.6% | Room temperature, 15.8 nm (95.0%), PdI: 0.211 | No obvious changes were observed. |
| 22 | 11.9 (91.9%) nm, PdI: 0.206 HPLC content: 98.3% | Room temperature, 12.4 (93.5%) nm, PdI: 0.215 | No obvious changes were observed. |
| 23 | 27.5 nm (77.9%), PdI: 0.328 HPLC content: 95.7% | 3-8 °C, 25.9 nm (89.4%), PdI: 0.245 | No obvious changes were observed. |
| 24 | 24.5 nm (85.5%), PdI: 0.253 HPLC content: 96.1% | 3-8 °C, 22.6 nm (91.0%), PdI: 0.302 | No obvious changes were observed. |
| 25 | 26.2 nm (75.2%), PdI: 0.325 HPLC content: 94.7% | 3-8 °C, 28.3 nm (86.1%), PdI: 0.264 | No obvious changes were observed. |
| 26 | 22.7 nm (83.4%), PdI: 0.372 HPLC content: 95.3% | 3-8 °C, 30.2 nm (79.3%), PdI: 0.427 | No obvious changes were observed. |
| 27 | 19.6 nm (75.9%), PdI: 0.424 HPLC content: 99.2% | Room temperature, 20.5 nm (87.2%), PdI: 0.341 | No obvious changes were observed. |
| 28 | 23.7 nm (84.2%), PdI: 0.323 HPLC content: 96.1% | Room temperature, 25.1 nm (82.7%), PdI: 0.294 | No obvious changes were observed. |
| 29 | 22.4 nm (91.4%), PdI: 0.293 HPLC content: 97.3% | Room temperature, 20.6 nm (93.6%), PdI: 0.258 | No obvious changes were observed. |
| 30 | 24.1 nm (81.5%), PdI: 0.357 HPLC content: 95.6% | Room temperature, 26.4 nm (84.2%), PdI: 0.263 | No obvious changes were observed. |
| 31 | 27.5 nm (79.6%), PdI: 0.364 HPLC content: 97.2% | Room temperature, 30.5 nm (81.6%), PdI: 0.407 | No obvious changes were observed. |
| 32 | 17.6 nm (93.9%), PdI: 0.229 HPLC content: 95.3% | Room temperature, 19.6 nm (89.2%), PdI: 0.317 | No obvious changes were observed. |
| 33 | 19.7 nm (86.4%), PdI: 0.331 HPLC content: 94.7% | Room temperature, 23.6 nm (78.2%), PdI: 0.410 | No obvious changes were observed. |
| 34 | 18.6 nm (96.9%), PdI: 0.257, HPLC content: 98.1% | Room temperature, 19.3 nm (95.2%), PdI: 0.226 | No obvious changes were observed. |
| 35 | 18.6 nm (96.5%), PdI: 0.218; HPLC content: 97.2% | Room temperature, 21.4 nm (93.5%), PdI: 0.269 | No obvious changes were observed. |
| 36 | 12.8 nm (82.8%), PdI: 0.372 HPLC content: N/A | Room temperature, 12.0 nm (72.7%), 453 nm (24.7%), PdI: 0.372 | No obvious changes were observed. |
| Comp arative examp le 1 | 10.0 nm (98.8%), PdI: 0.363 HPLC content: 99.3% | Room temperature | Precipitations were observed. |
| Comp arative examp le 2 | 196 nm (61.6%), PdI: 0.828 HPLC content: 94.7% | Room temperature, supernatant 530 nm (53.9%) and 225 nm (46.1%), PdI: 1.00 | Cottony precipitations were observed. |

**Table 5:**

| Exam ple | Initial particle size /PdI/ HPLC content | Storing in a dark place at a certain temperature for one month (unless otherwise stated) | |
|---|---|---|---|
| | | Particle size/PdI | Appearance and content changes |
| 37 | 14.0 nm (54.2%), 222 nm (31.9%) and 10.1 nm (13.1%), PdI: 0.564 HPLC content: 96.4% | Storing at 40 °C for 20 days, 242 nm (77.9%) and 18.1 nm (12.4%), PdI: 0.372 | No obvious changes were observed. |
| 38 | 15.1 nm (87.1%) and 3.1 nm (11.0%), PdI: 0.288 HPLC content: 98.1% | Room temperature, 17.0 nm (84.6%), PdI: 0.226 | No obvious changes were observed. |
| 39 | 11.5 nm (62.9%) and 77.8 nm (23.6%), PdI: 0.362 HPLC content: 99.3% | Room temperature, 16.2 nm (87.1%), PdI: 0.329 | No obvious changes were observed. |
| 40 | 125.6 nm (63.5%), 13.6 nm (33.1%), PdI: 0.255 HPLC content: 98.5% | Room temperature | Light emulsion with white precipitates formed. |
| Comp arative examp le 3 | 1299 nm, PdI: 0.175 HPLC content: unstable, not detected | Standing overnight at room temperature | White precipitates generated. |

According to the above results, the carrier or auxiliary material prepared in the present invention could successfully assemble various types of ophthalmic medicaments to prepare ophthalmic preparations. Moreover, the preparation had a small particle size, a high content of active pharmaceutical ingredients detected by HPLC, as well as the stable morphology and content after long-term storage; this indicated that the carrier or auxiliary material of the present invention had high encapsulation efficiency for ophthalmic medicaments and good stability. However, the preparation prepared in the Comparative example using different excipients and starting materials from the present invention had poor stability and might experience precipitation or change in a short period of time.

### Experimental example 3: Antiviral experiment of ophthalmic preparations prepared with the carrier or auxiliary material of the present invention

### 1. Experimental object: Eye drop delivery systems of ganciclovir prepared in Example 27.

### 2. Experimental method:

The eye drop delivery systems of ganciclovir prepared in the Examples were tested for its *in vitro* inhibitory acitivies against human cytomegalovirus (HCMV).

Human embryo lung fibroblasts (MRC-5) infected with human cytomegalovirus strains HCMV-AD169 were chosen and used in the experiment, and the antiviral activity of ganciclovir was investigated with half effective dose (EC50). Four groups were included in the experiment, that is, the test group of the eye drop delivery systems prepared in the Examples, the test group of ganciclovir technical drug, the negative control group (MRC-5 cells), and the positive control group (MRC-5 cells infected with HCMV-AD169). The samples of both test groups were diluted with the culture media to obtain 6 diluted concentrations: 500 µg/mL, 250 µg/mL, 125 µg/mL, 62.5 µg/mL, 31.25 µg/mL, and 15.63 µg/mL, and 4 wells were set for each diluted concentration. Cytopathic effect (CPE) was used as an indicator: 0 for no cytopathy, 1 for ≤25% cytopathy, 2 for 25%-50% cytopathy, 3 for 50%-75% cytopathy, and 4 for 75%-100% cytopathy. The half effective dose (EC50) was calculated using Reed-Munch method: EC50 = 10n; n = [(logarithm of the dilution degree corresponding to an efficiency of >50%)+(percentage of >50% efficiency - 50%)/(percentage of >50% efficiency - percentage of <50% efficiency) × logarithm of the dilution coefficient.

The experimental procedures were as follows: MRC-5 cells were adjusting to approximate 1.5×10⁵/mL, and then added a 96 well plate, 100 µL of culture media for each well. The plate was cultured in a 37 °C, 5% CO₂ cell incubator, and then the supernatant was discarded after the cells adhered to the wall as a monolayer. Except for the control groups, cells were added to each well of both test groups at a concention of 100× TC50 (half of the virus infected cells obtained in the pre-experiment) and then incubated for 2 h. The supernatant was discarded. A series of test samples were added at 100 µL/well, and then the plate was further cultivated. The CPE of each well was observed. When the CPE of the positive control group reached more than 90%, the CPE of each well was recorded, and the half effective dose (EC50) was calculated using the Reed Munch method.

### 3. Experimental results: The results are shown in Table 6.

**Table 6. Inhibition results of test samples against HCMV-AD169 virus.**

| Group | Concentration (µg/mL) | CPE of each well | | | | Effective rate (%) |
|---|---|---|---|---|---|---|
| Ganciclovir technical drug | 500 | 0 | 1 | 0 | 0 | 97 |
| | 250 | 1 | 1 | 1 | 0 | 91 |
| | 125 | 2 | 2 | 1 | 2 | 77 |
| | 62.5 | 2 | 3 | 3 | 3 | 64 |
| | 31.25 | 3 | 3 | 3 | 3 | 35 |
| | 15.63 | 4 | 3 | 3 | 3 | 21 |
| Eye drop delivery systems of ganciclovir | 500 | 0 | 0 | 0 | 1 | 97 |
| | 250 | 1 | 1 | 0 | 1 | 91 |
| | 125 | 1 | 2 | 2 | 2 | 77 |
| | 62.5 | 3 | 2 | 3 | 3 | 64 |
| | 31.25 | 3 | 3 | 3 | 3 | 35 |
| | 15.63 | 4 | 3 | 3 | 3 | 21 |
| Positive control | / | 4 | 4 | 4 | 4 | / |
| Negative control | / | 0 | 0 | 0 | 0 | / |

Conclusion: The eye drop delivery system of ganciclovir prepared in Example 27 has no difference in inhibiting the virus *in vitro,* compared with the ganciclovir technical drug.

The above results indicated that the carrier or auxiliary material of the ophthalmic preparation according to the present invention, as a drug carrier for eye drops, would not affect the efficacy of the active pharmaceutical ingredients carried (encapsulated) for treating eye diseases.

### Experimental example 4: In vitro bacteriostatic test of ophthalmic preparations prepared with the carrier or auxiliary material of the present invention

### 1. Experimental object: Eye drop delivery systems of moxifloxacin prepared in Example 3.

### 1.1 Experimental method:

The *in vitro* bacteriostasis test (MIC) was carried out on the moxifloxacin eye drop delivery system prepared in the Example. The moxifloxacin technical drug was used as the control, while the moxifloxacin eye drop delivery system prepared was used as the test sample, to prepare a drug sensitive plate, so that the drug sensitive plate contained 7-8 diluted concentrations of moxifloxacin technical drug (the control) and the test sample, which was obtained by dilution in a ratio of 1:2. 25 bacterial strains incubated for 18 h-24 h were chosen using inoculation loop, and transferred into sterile physiological saline to form a bacterial suspension equivalent to 0.5 MCF; the bacterial suspension was added to the liquid drug sensitive media, in which the ratio of the bacterial suspension to the liquid media was 1:200, and mixed well, then 100 µL of diluted bacterial solution was added to each well; the media was incubated at 35 °C ± 2 °C for 16-20 h; the growth of bacteria in each well was determined by turbidity, and observed from low concentration to high concentration. The minimum drug concentration that could inhibit bacterial growth was the MIC of the drug.

### 1.2 Experimental results: The results are shown in Table 7.

**Table 7. Test Results of moxifloxacin eye drop delivery system using drug sensitive plate (broth microdilution method).**

| Bacterial strains | Minimum inhibitory concentration (MIC, µg/mL) | |
|---|---|---|
| | Moxifloxacin (control) | Test sample |
| *Escherichia coli* ATCC25922 | 0.06 | 0.06 |
| *Staphylococcus aureus* ATCC29213 | 0.12 | 0.12 |
| *Pseudomonas aeruginosa* ATCC27853 | 4 | 1 |
| *Enterococcus faecalis* ATCC29212 | 0.5 | 0.25 |
| *Elaemophilus influenza* ATCC49247 | <0.032 | <0.032 |
| *Streptococcus pneumoniae* ATCC49619 | 0.12 | 0.12 |
| *Staphylococcus aureus* | 0.12 | 0.06 |
| *Staphylococcus aureus* | 0.12 | 0.12 |
| *Staphylococcus aureus* | 0.12 | 0.12 |
| *Staphylococcus aureus* | 0.12 | 0.12 |
| *Staphylococcus aureus* | 0.12 | 0.12 |
| *Staphylococcus aureus* | 0.12 | 0.12 |
| *Staphylococcus aureus* | 0.12 | 0.12 |
| *Staphylococcus aureus* | 0.12 | 0.12 |
| *Staphylococcus aureus* | 0.12 | 0.12 |
| *Staphylococcus aureus* | 0.12 | 0.12 |
| *Staphylococcus aureus* | 0.25 | 0.25 |
| *Staphylococcus aureus* | 0.25 | 0.25 |
| Group A Streptococcus | 0.12 | 0.12 |
| Group A Streptococcus | 0.25 | 0.5 |
| Group A Streptococcus | 0.25 | 0.25 |
| Group A Streptococcus | 0.25 | 0.25 |
| *Streptococcus pneumoniae* | 0.25 | 0.25 |
| *Streptococcus pneumoniae* | 0.25 | 0.25 |
| *Streptococcus pneumoniae* | 0.25 | 0.25 |

Conclusion: compared with the control, the moxifloxacin eye drop system prepared in Example 3 did not show significant difference in the bacteriostatic effect.

Based on the above results, the carrier or auxiliary material of the ophthalmic preparation according to the present invention, as an eye drop delivery carrier, would not affect the properties and effects of the active pharmaceutical ingredients carried (encapsulated) for treating eye diseases.

In summary, the present invention provided a carrier or auxiliary material of an ophthalmic preparation, as well as an application thereof. The carrier or auxiliary material of the ophthalmic preparation according to the present invention, as an eye drop delivery carrier, would not affect the properties and effects of the active pharmaceutical ingredients carried (encapsulated) for treating eye diseases, could wrap a drug through an anterior segment of the eye, and efficiently deliver the drug to a posterior segment, to play a therapeutic action, therby achieving the object of treating ocular fundus diseases by means of eye drop administration, solving the technical problem that were expected to be urgently settled but had not been resolved in the field of ophthalmic drug delivery, and presenting extremely excellent clinical application prospects and very positive social significance.

## Claims

1. A carrier or auxiliary material of an ophthalmic preparation, **characterized in that** it contains the following components: a surfactant and an ionic polymer, and it also contains a solvent.

2. The carrier or auxiliary material of an ophthalmic preparation according to claim 1, **characterized in that** the mass ratio of the surfactant to the ionic polymer is (1-100):(0.1-50); the ratio of the surfactant to the solvent is: every 100 mL of the solvent contains 5-3000 mg of the surfactant;
preferably, the mass ratio of the surfactant to the ionic polymer is (1-31):(1-7.5); the ratio of the surfactant to the solvent is: every 100 mL of the solvent contains 50-3000 mg of the surfactant.

3. The carrier or auxiliary material of an ophthalmic preparation according to claim 1 or 2, **characterized in that** the surfactant is a non-ionic surfactant.

4. The carrier or auxiliary material of an ophthalmic preparation according to claim 3, **characterized in that** the non-ionic surfactant is Spans, Polysorbates, Poloxamer, alkylglucosides, vitamin E polyethylene glycol succinate (TPGS), sucrose stearates or azone.

5. The carrier or auxiliary material of an ophthalmic preparation according to claim 1 or 2, **characterized in that** the ionic polymer is selected from at least one of carboxymethyl cellulose (CMC) and its salts, sodium starch glycolate, hyaluronic acid and its salts, Xanthan gum, alginic acid and its salts, and polyethylene glycol diacetate PEG-(COOH)₂.

6. A carrier or auxiliary material of an ophthalmic preparation, **characterized in that** it contains the following components: a low-polymerization-degree povidone and a medium-polymerization-degree povidone, and it also contains a solvent.

7. The carrier or auxiliary material of an ophthalmic preparation according to claim 6, **characterized in that** the mass ratio of the low-polymerization-degree povidone to the medium-polymerization-degree povidone is (0.1-10):1, and the ratio of the low-polymerization-degree povidone to the solvent is: every 100 mL of the solvent contains 5-3000 mg of the low-polymerization-degree povidone;
preferably, the mass ratio of the low-polymerization-degree povidone to the medium-polymerization-degree povidone is (0.24-1):1, and the ratio of the low-polymerization-degree povidone to the solvent is: every 100 mL of the solvent contains 400-840 mg of the low-polymerization-degree povidone.

8. The carrier or auxiliary material of an ophthalmic preparation according to claim 6 or 7, **characterized in that** the low-polymerization-degree povidone is a povidone with a weight average molecular weight of 2000-5000 Dalton, and the medium-polymerization-degree povidone is a povidone with a weight average molecular weight of 20000-60000 Dalton.

9. The carrier or auxiliary material of an ophthalmic preparation according to claim 6 or 7, **characterized in that** the low-polymerization-degree povidone is a povidone PVP K12 with a weight average molecular weight of 3500 Dalton, and the medium-polymerization-degree povidone is a povidone PVP K30 with a weight average molecular weight of 35000-50000 Dalton.

10. The carrier or auxiliary material of an ophthalmic preparation according to any one of claims 1 to 9, **characterized in that** the solvent is a polar solvent.

11. The carrier or auxiliary material of an ophthalmic preparation according to claim 10, **characterized in that** the polar solvent is water.

12. The carrier or auxiliary material of an ophthalmic preparation according to any one of claims 1 to 11, **characterized in that** it also contains the following components: adhesive agents and/or cosolvents;
preferably, the adhesive agent is selected from at least one of polyethylene glycol, carbomer, Poloxamer, povidone, hydroxypropyl cellulose, hydroxyethyl cellulose, methyl cellulose, polyvinyl alcohol, Xanthan gum, polyoxyethylene fatty alcohols, hyaluronic acid and its salts or hydroxypropyl methyl cellulose (HPMC); the cosolvent is propylene glycol, glycerol, liquid polyethylene glycol, hydrogenated castor oil or castor oil; the mass ratio of the adhesive agent to the surfactant is 1:(0.1-100), and the mass ratio of the cosolvent to the surfactant is (1-10):1; the mass ratio of the adhesive agent to the low-polymerization-degree povidone is 1:(0.1-100), and the mass ratio of the cosolvent to the low-polymerization-degree povidone is (1-10):1;
more preferably, the mass ratio of the adhesive agent to the surfactant is 1:(0.1-30); the mass ratio of the adhesive agent to the low-polymerization-degree povidone is 1:(0.1-30).

13. The carrier or auxiliary material of an ophthalmic preparation according to any one of claims 1 to 12, **characterized in that** it also contains nanobodies, which are spherical and have a particle size of 1-100 nm; the nanobodies are formed by self-assembly of the components contained in the carrier or auxiliary material of the ophthalmic preparation.

14. The carrier or auxiliary material of an ophthalmic preparation according to claim 13, **characterized in that** the nanobodies have a particle size of 5-30 nm.

15. The carrier or auxiliary material of an ophthalmic preparation according to claim 13 or 14, **characterized in that** it contains nanospheres, which are spherical in shape and have a particle size of 10-2000 nm; the nanospheres are formed by self-assembly of nanobodies.

16. The carrier or auxiliary material of an ophthalmic preparation according to claim 15, **characterized in that** the particle size of the nanospheres is 100-2000 nm.

17. A method for preparing the carrier or auxiliary material of an ophthalmic preparation according to any one of claims 1 to 16, **characterized in that** it involves uniformly mixing the component and the solvent into a solution, and then the solution is ground or uniformly dispersed.

18. The carrier or auxiliary material of an ophthalmic preparation according to any one of claims 1 to 16 for use in the preparation of carriers for eye drop administration, which can deliver the medicament to the posterior segment of eyes.

19. An ophthalmic preparation for eye drop administration, **characterized in that** it is a preparation composed of the carrier or auxiliary material of an ophthalmic preparation according to any one of claims 1 to 16 as well as an active pharmaceutical ingredient for treating eye diseases.

20. The ophthalmic preparation according to claim 19, **characterized in that** the mass ratio of the surfactant in the carrier or auxiliary material of an ophthalmic preparation to the active pharmaceutical ingredient for treating eye diseases is (1-30):(1-2);
or, the mass ratio of the low-polymerization-degree povidone in the carrier or auxiliary material of an ophthalmic preparation to the active pharmaceutical ingredient for treating eye diseases is (6-40): 1.

21. The ophthalmic preparation according to claim 19, **characterized in that** the ophthalmic preparation carrier or auxiliary material contains nanobodies, and the nanobodies are assembled with the active pharmaceutical ingredient for treating eye diseases.

22. The ophthalmic preparation according to claim 21, **characterized in that** the nanobody is spherical, with a particle size of 1-100 nm; preferably, the particle size is 5-30 nm.

23. The ophthalmic preparation according to any one of claims 19 to 22, **characterized in that** the active pharmaceutical ingredient for treating eye diseases include small molecule compound medicaments, or free acids thereof, or free bases thereof, or pharmaceutically acceptable salts thereof;
preferably, the small molecule compound medicaments include nucleoside antiviral medicaments, intraocular pressure (IOP) lowering medicaments, antibiotics, antioxidant medicaments, anti-inflammatory medicaments, muscarinic receptor blocker medicaments, immunosuppressive medicaments, and glucocorticoid medicaments;
more preferably, the nucleoside antiviral medicaments include ganciclovir, aciclovir, penciclovir, cidofovir, fomivirsen, and lobucavir;
the IOP-lowering medicaments include a carbonic anhydrase inhibitor, and more preferably, are brinzolamide, acetazolamide, and methazolamide;
the antibiotics include amikacin, ceftriaxone, cefazolin, oxacillin, levofloxacin, ciprofloxacin, moxifloxacin, and vancomycin;
the anti-inflammatory medicament includes oxytetracycline;
the antioxidants include taurine, anthocyanidin, and lignin;
the muscarinic receptor blocker medicaments include atropine, scopolamine, and anisodamine;
the immunosuppressive medicaments include cyclosporine, tacrolimus, sirolimus, everolimus, mycophenolate mofetil, methotrexate, azathioprine, and cyclophosphamide;
the glucocorticoid medicaments include cortisone, prednisone, prednisolone, methylprednisolone, triamcinolone, and triamcinolone acetonide.

24. A method for preparing the ophthalmic preparation according to any one of claims 19 to 23, **characterized in that** it comprises the following steps:
(1) The surfactant and/or the adhesive agent is added to the solvent to prepare a solution;
(2) The active pharmaceutical ingredient for treating eye diseases and/or the cosolvent is dispersed in the solution obtained in step (1), to which is then added the ionic polymer or its solution, and the resultant solution is dispersed and mixed to obtain the initial suspension;
(3) The initial suspension obtained in step (2) is stirred and dispersed or homogenized to obtain the preparation;
or comprises the following steps:
(a) The low-polymerization-degree povidone and/or the adhesive agent is added to the solvent to prepare a solution;
(b) The active pharmaceutical ingredient for treating eye diseases and/or the cosolvent is dispersed in the solution obtained in step (a), to which is then added the medium-polymerization-degree povidone or its solution, and the resultant solution is dispersed and mixed to obtain the initial suspension;
(c) The mixed solution obtained in step (b) is ground or uniformly dispersed to obtain the preparation.

25. The preparation method according to claim 24, **characterized in that** the dispersion described in step (2) or step (b) is selected from at least one of mechanical stirring dispersion, magnetic stirring dispersion, vortex shaking dispersion, shear dispersion, homogeneous dispersion, grinding dispersion, and ultrasonic dispersion.

26. The ophthalmic preparation according to any one of claims 19 to 23 for use in the preparation of medicaments for treating eye diseases.

27. The use according to claim 26, **characterized in that** the ophthalmic preparation is an ophthalmic preparation for treating fundus diseases, and/or treating viral infectious diseases in the posterior segment of the eye, and/or treating chronic inflammation in the posterior segment of the eye, and/or reducing intraocular pressure, and/or relieving eye pain, and/or treating ocular bacterial or fungal infections, and/or preventing and treating juvenile myopia and pseudomyopia, and/or treating autoimmune diseases, and/or treating anterior segment diseases of the eye, and/or inhibiting tumor growth.

28. The use according to claim 27, **characterized in that**:
the ophthalmic preparation for treating fundus diseases includes an ophthalmic preparation for treating macular edema, inflammatory edema, and inflammatory pain caused by fundus vascular diseases; more preferably, includes an ophthalmic preparation for treating macular edema due to central retinal vein occlusion, macular edema due to branch retinal vein occlusion, diabetic retinopathy (DR), diabetic macular edema (DME), pathological myopic macular edema, macular edema due to wet age-related macular degeneration, dry macular degeneration, geographic atrophy, endophthalmitis, acute retinal necrosis, postoperative inflammatory pain, and uveitis;
the ophthalmic preparation for treating viral infectious diseases in the posterior segment of the eye includes an ophthalmic preparation for treating cytomegaloviral uveitis, viral optic neuritis, and viral acute retinal necrosis;
the ophthalmic preparation for reducing intraocular pressure includes an ophthalmic preparation for treating acute and chronic glaucoma and its complications;
the ophthalmic preparation for treating autoimmune diseases includes an ophthalmic preparation for treating the eye diseases caused by ocular immune diseases (OID) or systemic autoimmune diseases; preferably, includes an ophthalmic preparation for treating Graves ophthalmopathy, Behoet's syndrome, birdshot retina choroidopathy, Sjogren's syndrome, sympathetic ophthalmia or granulomatous eye disease;
the ophthalmic preparation for treating anterior segment diseases of the eye includes an ophthalmic preparation for treating postoperative sequelae or complications of high-risk corneal transplantation, Vernal Kerato-Conjunctivits (VKC), Mooren's ulcer or spontaneous chronic corneal epithelial defects (SCCEDs), herpes simplex keratitis (HSK), corneal neovascularization or corneal pterygium.

29. The use according to claim 27, **characterized in that** the active pharmaceutical ingredient of the ophthalmic preparation for treating intraocular bacterial or fungal infections is antibiotics;
the active pharmaceutical ingredient of the ophthalmic preparation for preventing and treating juvenile myopia and pseudomyopia is muscarinic receptor blockers;
the active pharmaceutical ingredient of the ophthalmic preparation for treating autoimmune diseases is mmunosuppressive agents.

30. A method for treating eye diseases, **characterized in that** the ophthalmic preparation according to any one of claims 19 to 23 is administrated to the patients.

31. The method according to claim 30, **characterized in that** the eye diseases are fundus diseases, and/or viral infectious diseases in the posterior segment of the eye, and/or chronic inflammation in the posterior segment of the eye, and/or ocular hypertension, and/or eye pain, and/or ocular bacterial or fungal infections, and/or juvenile myopia and pseudomyopia, and/or autoimmune diseases, and/or anterior segment diseases of the eye, and/or ocular tumors.

32. The method according to claim 31, **characterized in that** the fundus diseases includes macular edema, inflammatory edema, and inflammatory pain, that are caused by fundus vascular diseases; more preferably, are macular edema due to central retinal vein occlusion, macular edema due to branch retinal vein occlusion, diabetic retinopathy (DR), diabetic macular edema (DME), pathological myopic macular edema, macular edema due to wet age-related macular degeneration, dry macular degeneration, geographic atrophy, endophthalmitis, acute retinal necrosis, postoperative inflammatory pain, and uveitis;
the viral infectious diseases in the posterior segment of the eye include cytomegaloviral uveitis, viral optic neuritis, and viral acute retinal necrosis;
the ocular hypertension includes acute and chronic glaucoma and its complications;
the autoimmune diseases include the eye diseases caused by ocular immune diseases (OID) or systemic autoimmune diseases; preferably, include Graves ophthalmopathy, Behoet's syndrome, birdshot retina choroidopathy, Sjogren's syndrome, sympathetic ophthalmia or granulomatous eye disease;
the anterior segment diseases of the eye include postoperative sequelae or complications of high-risk corneal transplantation, Vernal Kerato-Conjunctivits (VKC), Mooren's ulcer or spontaneous chronic corneal epithelial defects (SCCEDs), herpes simplex keratitis (HSK), corneal neovascularization or corneal pterygium.

33. The method according to claim 30, **characterized in that** the way used is eye drop administration.
